# EUROPÄISCHE PATENTSCHRIFT

(11) **EP 2 525 656 B1**
(45) Veröffentlichungstag und Bekanntmachung des Hinweises auf die Patenterteilung: **28.12.2016**
(21) Anmeldenummer: 11700138.8
(22) Anmeldetag: 13.01.2011
(51) Int. Cl.: A01N 25/30, C07C 215/12, A01N 25/00, A01N 57/20, A01N 43/50, A01N 33/12

(54) **ZUSAMMENSETZUNG UMFASSEND EIN PESTIZID UND EIN ALKOXYLAT VON 2-PROPYLHEPTYLAMIN**
COMPOUND COMPRISING A PESTICIDE AND AN ALKOXYLATE OF 2-PROPYLHEPTYL AMINE
COMPOSITION COMPRENANT UN PESTICIDE ET UN ALCOXYLATE DE 2-PROPYLHEPTYLAMINE

(30) Priorität: 23.03.2010 EP 10157267; 18.01.2010 EP 10150986; 18.01.2010 US 295784 P
(43) Veröffentlichungstag der Anmeldung: 28.11.2012
(73) Patentinhaber: BASF Corporation, Florham Park, NJ 07932 (US)
(72) Erfinder: KLINGELHOEFER, Paul, 68165 Mannheim (DE); KINGMA, Arend, Jouke, 67069 Ludwigshafen (DE); MAITRO-VOGEL, Sophie, 68199 Mannheim (DE); HUYGHE, Kevin, 2950 Kapellen (BE); HADERLEIN, Gerd, 67269 Grünstadt (DE); SCHNABEL, Gerhard, 63820 Elsenfeld (DE); NOLTE, Marc, 68165 Mannheim (DE); EVANS, Richard, Roger, 67117 Limburgerhof (DE)
(74) Vertreter: BASF IP Association
(86) Internationale Anmeldenummer: PCT/EP2011/050369
(87) Internationale Veröffentlichungsnummer: WO 2011/086115

(56) Entgegenhaltungen:
- EP-A1- 0 696 572
- WO-A1-2006/034459
- WO-A1-2009/004044
- WO-A2-2007/030312
- US-A- 5 668 085
- US-A1- 2006 019 828
- US-A1- 2011 315 910

## Beschreibung

Gegenstand der vorliegenden Erfindung ist eine Zusammensetzung umfassend ein Pestizid und ein Alkoxylat. Außerdem betrifft die Erfindung das Alkoxylat, ein Verfahren zu seiner Herstellung, und seine Verwendung als Hilfsmittel in Pestizid-haltigen Spritzbrühen. Die Erfindung betrifft weiterhin ein Verfahren zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt. Ferner betrifft die Erfindung Saatgut enthaltend die Zusammensetzung. Kombinationen bevorzugter Merkmale mit anderen bevorzugten Merkmalen werden von der vorliegenden Erfindung umfasst.

Alkoxylate und ihre Verwendung in agrochemischen Formulierungen als Adjuvantien sind allgemein bekannt:

WO 2009/004044 offenbart eine herbizide Zusammensetzung umfassend ein Phenoxysäure Herbizid und ein alkoxyliertes Alkylamin als Adjuvants, wobei das Alkylamin beispielsweise ein 2-Propylheptyl-methylamin sein kann, das mit 3 bis 20 Gruppen Ethylenoxid alkoxyliert ist.

US 5,668,085 offenbart eine herbizide Zusammensetzung umfassend eine wässrige Lösung von Glyphosat und Tensid. Das Tensid kann ein alkoxyliertes Alkylamin sein, wobei die Alkylgruppe 8 bis 22 Kohlenstoffatome enthält.

Alkoxylierte Alkylamine, insbesondere kommerziell erhältliche ethoxylierte Talgfettamine (POEA), haben kritische toxische (wie Haut- und Augenreizung) und ökotoxische Eigenschaften (wie hohe Ecotoxizität gegen Wasserorganismen wie Algen und Daphnien). So gilt beispielsweise das in Roundup® Herbiziden oft als Netzmittel enthaltene POEA (CAS Nr. 61791-26-2) als verhältnismäßig aquatoxisch (Tsui und Chu, Chemosphere 2003, 52, 1189-1197).

Aufgabe der vorliegende Erfindung war daher ein Adjuvants zu finden, das für Herbizide wie Glyphosat gut geeignet ist und dabei weniger toxisch (v.a. weniger aquatoxisch) ist. Weiterhin sollte das Adjuvants eine lagerstabile Formulierung der Pestizide ermöglichen.

Die Aufgabe wurde gelöst durch eine Zusammensetzung umfassend ein Pestizid und ein Alkoxylat, wobei das Alkoxylat ein Aminalkoxylat (A) 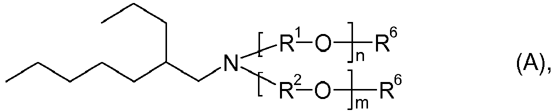 oder ein quartärnisiertes Derivat (AQ) 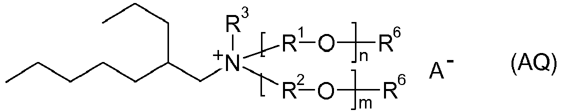 des Aminalkoxylats (A) ist, wobei
R¹, R², und R⁵ unabhängig voneinander Ethylen, Propylen, Butylen oder eine Mischung davon sind,
R³ ein H, -OH, -OR⁴, -[R⁵-O]ₚ-R⁶, C₁-C₆ Alkyl oder ein Sauerstoffanion,
R⁴ ein C₁-C₆ Alkyl, C₂-C₆ Alkenyl, oder C₂-C₆ Alkinyl,
R⁶ ein H, C₁-C₆ Alkyl, C₂-C₆ Alkenyl, C₂-C₆ Alkinyl, -SO₃R^{a}, -P(O)OR^{b}OR^{c}, -CH₂CO₂R^{d}, oder -C(O)R^{e},
R^{a} und R^{d} unabhängig voneinander ein H, anorganische oder organische Kationen,
R^{b} und R^{c} unabhängig voneinander ein H, anorganische oder organische Kationen, C₁-C₆ Alkyl, C₂-C₆ Alkenyl oder C₂-C₆ Alkinyl,
R^{e} C₁-C₂₂-Alkyl, C₂-C₂₂-Alkenyl, C₂-C₂₂-Alkinyl, C₆-C₂₂-Aryl, C₇-C₂₂-Alkylaryl,
n, m und p unabhängig voneinander ein Wert von 1 bis 30,
A- ein landwirtschaftlich akzeptables Anion, oder, falls R³ ein Sauerstoffanion ist, A-nicht vorhanden ist.

Bevorzugt umfasst die erfindungsgemäße Zusammensetzung ein Pestizid und ein Alkoxylat, wobei das Alkoxylat ein Aminalkoxylat (A) ist.

Bevorzugt hat n ein Wert von 1 bis 20, besonders bevorzugt von 1 bis 15. Bevorzugt hat m ein Wert von 1 bis 20, besonders bevorzugt von 1 bis 15. Bevorzugt hat p ein Wert von 1 bis 30, besonders bevorzugt von 1 bis 20. Die Werte von n, m und o sind normalerweise durchnittliche Werte, wie sie meist bei der Alkoxylierung mit Alkoxiden entstehen. Daher können n, m und o sowohl ganzzahlige Werte als auch alle Werte zwischen den ganzen Zahlen einnehmen.

Bevorzugt ist beim Aminalkoxlat (A) ist die Summe aus n und m 2 bis 40, und bei dessen quartärnisiertes Derivat (AQ) ist die Summe aus n, m und p 3 bis 80.

Beim Aminalkoxlat (A) ist die Summe aus n und m ist besonders bevorzugt 3 bis 30, und speziell 5 bis 25. In einer weiteren speziell bevorzugten Ausführungsform ist die Summe aus n und m 6 bis 9, insbesondere 6,5 bis 8,5 und insbesondere 6,9 bis 7,9. In einer weiteren speziell bevorzugten Ausführungsform ist die Summe aus n und m 11 bis 40, insbesondere 12 bis 30 und insbesondere 13,5 bis 25. In einer weiteren speziell bevorzugten Ausführungsform ist die Summe aus n und m 8 bis 13, insbesondere 9 bis 11.
Beim quartärnisierten Derivat (AQ) des Aminalkoxlat (A) ist die Summe aus n, m und p besonders bevorzugt 3 bis 40, und speziell 5 bis 25. In einer speziell bevorzugten Ausführungsform ist die Summe aus n und m 8 bis 13, insbesondere 9 bis 11.

R¹, R² und R⁵ sind bevorzugt unabhängig voneinander Ethylen, Ethylen und Propylen, Ethylen und Butylen, oder Ethylen, Propylen und Butylen. In einer weiteren bevorzugten Ausführungsform sind R¹, R² und R⁵ Propylen. In einer weiteren bevorzugten Ausführungsform sind R¹, R² und R⁵ Butylen. Besonders bevorzugt sind R¹, R² und R⁵ unabhängig voneinander Ethylen, oder Ethylen und Propylen. Ganz besonders bevorzugt sind R¹, R² und R⁵ Ethylen.

Wenn R¹, R² oder R⁵ eine Butylenrest enthalten, kann dieser als n-Butylen, iso-Butylen, oder 2,3-Butylengruppe vorliegen, wobei n-Butylen und iso-Butylen bevorzugt sind und n-Butylen am meisten bevorzugt ist.

R¹, R², und R⁵ können unabhängig voneinander eine Mischung von Ethylen, Propylen, oder Butylen sein. Dabei kann zum Beispiel einer oder alle Reste R¹, R², und R⁵ jeweils in einer Alkoxlyatkette eine Mischung dieser Gruppen enthalten. Solche Mischungen können in beliebiger Reihenfolge miteinander verbunden sein, beispielsweise statistisch oder in Blockweise (wie ein Block Ethylen und ein Block Propylen). Es können auch jeweils einer oder mehrere der Reste R¹, R², und R⁵ eine komplette Alkoxlyatkette aus verschiedenen Alkylengruppen bilden. Beispielsweise kann R¹ und R² aus Ethylen und R⁵ aus Propylen aufgebaut sein.

R³ ist bevorzugt ein H, -OH, ₁-C₄ Alkyl oder ein Sauerstoffanion, besonders bevorzugt ist es ein H, Methyl, Butyl oder ein Sauerstoffanion. In einer speziell bevorzugten Ausführungsform ist R³ ein Methyl. In einer weiteren speziell bevorzugten Ausführungsform ist R³ ein Sauerstoffanion. In einer weiteren speziell bevorzugten Ausführungsform ist R³ ein H.

R⁴ ist bevorzugt ein C₁-C₆ Alkyl, insbesondere ein Methyl oder Butyl, speziell Methyl.

R⁶ ist bevorzugt ein H oder C₁-C₆ Alkyl, besonders bevorzugt ein H oder Methyl, insbesondere H.

R^{a} und R^{d} sind unabhängig voneinander H, oder anorganische oder organische Kationen, welche einfach oder mehrfach positiv geladen sein können. Beispiele für anorganische Kationen sind Kationen von Ammonium, Na⁺, K⁺, Mg²⁺, Ca²⁺, oder Zn²⁺. Beispiele für organische Kationen sindMethylammonium, Dimethylammonium, Trimethylammonium, Tetramethylammonium, (2-Hydroxyethyl)ammonium, Bis(2-hydroxyethyl)-ammonium, Tris(2-hydroxyethyl)ammonium, Tetra(2-hydroxyethyl)ammonium. Bevorzugt sind R^{a} und R^{d} sind unabhängig voneinander H, oder anorganische Kationen. Wenn ein anorganisches oder organisches Kation vorliegt, dann würde die zugehörige anionische Gruppe von der entsprechenden funktionellen Guppe (z.B. -SO₃⁻, -P(O)O-O-, oder -CH₂CO₂⁻) an R⁶ gebildet werden.

R^{b} und R^{c} sind bevorzugt unabhängig voneinander H, anorganische oder organische Kationen. Geeignete anorganische oder organische Kationen sind die unter R^{a} genannten.

In einer weiteren Ausführungsform können beim quarternären Derivat (AQ) die Reste R^{a}, R^{b}, R^{c} und R^{d} unabhängig voneinander organische Kationen sein, wobei die kationische Gruppe das quarternäre Stickstoff-Kation von AQ selber ist. Dabei könnte AQ also ein Zwitterion bilden, wobei die anionische Gruppe von der entsprechenden funktionellen Gruppe (z.B. -SO₃-, -P(O)O-O-, oder -CH₂CO₂⁻) an R⁶ in AQ gebildet wird, und die kationische Gruppe von dem quarternären Stickstoff von AQ. In dieser Zwitterionenform von AQ ist die Anwesenheit eines landwirtschaftlich akzeptablen Anions A-optional.
Re ist bevorzugt C₁-C₁₂-Alkyl, C₆-C₁₂-Aryl, oder C₇-C₁₂-Alkylaryl, besonders bevorzugt C₁-C₆-Alkyl.

A- ist ein landwirtschaftlich akzeptables Anion, wie sie dem Fachmann allgemein bekannt sind. Bevorzugt ist A- ein Halogenid (wie Chlorid oder Bromid), Phosphat, Sulfat oder ein anionisches Pestizid. Auch Carboxylate, wie Propionat, Acetat, Carbonat oder Formiat sind als A⁻ geeignet. Besonders bevorzugt ist A⁻ ein anionisches Pestizid, wie Glyphosatanion oder Glufosinatanion. Falls R³ ein Sauerstoffanion ist, liegt ein Aminoxid vor. In diesem Fall ist ein weiteres Anion wie A⁻ nicht vorhanden.

Beim Aminalkoxylat (A) sind bevorzugt R¹ und R² unabhängig voneinander Ethylen, Ethylen und Propylen, Ethylen und Butylen, oder Ethylen, Propylen und Butylen, und die Summe aus n und m ist 2 bis 60, bevorzugt 2 bis 40, besonders bevorzugt 3 bis 30, und insbesondere 5 bis 25. In einer weiteren bevorzugten Ausführungsform ist R¹ und R² Ethylen, Ethylen und Propylen, Ethylen und Butylen, oder Ethylen, Propylen und Butylen und die Summe aus n und m 6 bis 9, insbesondere 6,5 bis 8,5 und insbesondere 6,9 bis 7,9. In einer weiteren bevorzugten Ausführungsform ist R¹ und R² Ethylen, Ethylen und Propylen, Ethylen und Butylen, oder Ethylen, Propylen und Butylen und die Summe aus n und m 11 bis 40, insbesondere 12 bis 30 und insbesondere 13,5 bis 25.In einer besonders bevorzugten Ausführungsform ist R¹ und R² Ethylen, Ethylen und Propylen, Ethylen und Butylen, oder Ethylen, Propylen und Butylen und die Summe aus n und m 6 bis 14, insbesondere 8 bis 12 und insbesondere 9 bis 11.

Beim Aminalkoxylat (A) sind besonders bevorzugt R¹ und R² Ethylen, und die Summe aus n und m ist 2 bis 60, bevorzugt 2 bis 40, besonders bevorzugt 3 bis 30, und insbesondere 5 bis 25. In einer weiteren besonders bevorzugten Ausführungsform ist R¹ und R² Ethylen und die Summe aus n und m 6 bis 9, insbesondere 6,5 bis 8,5 und insbesondere 6,9 bis 7,9. In einer weiteren besonders bevorzugten Ausführungsform ist R¹ und R² Ethylen und die Summe aus n und m 11 bis 40, insbesondere 12 bis 30 und insbesondere 13,5 bis 25.

Die Verbindungen (A) und (AQ) können als Gemische von Stereoisomeren vorliegen oder als isolierte Stereoisomere. Tautomere und Betaine sind von den Strukturen (A) und (AQ) ebenfalls umfasst.

Die erfindungsgemäße Zusammensetzung enthält meist 0,1 bis 90 Gew.% des Alkoxylats, bevorzugt 1 bis 50 Gew.% und insbesondere 3 bis 30 Gew.%.

Der Begriff Pestizide bezeichnet mindestens einen Wirkstoff ausgewählt aus der Gruppe der Fungizide, Insektizide, Nematizide, Herbizide, Safener, Molluskizide, Rodentizide und/oder Wachstumsregulatoren. Bevorzugte Pestizide sind Fungizide, Insektizide, Herbizide, und Wachstumsregulatoren. Besonders bevorzugte Pestizide sind Herbizide und Wachstumsregulatoren. Auch Mischungen von Pestiziden aus zwei oder mehr der vorgenannten Klassen können verwendet werden. Der Fachmann ist vertraut mit solchen Pestiziden, die beispielsweise in Pesticide Manual, 14th Ed. (2006), The British Crop Protection Council, London, gefunden werden können. Geeignete Pestizide sind :
A) Strobilurine:
   Azoxystrobin, Dimoxystrobin, Coumoxystrobin, Coumethoxystrobin, Enestroburin, Fluoxastrobin, Kresoxim-methyl, Metominostrobin, Orysastrobin, Picoxystrobin, Pyraclostrobin, Pyrametostrobin, Pyraoxystrobin, Pyribencarb, Trifloxystrobin, 2-[2-(2,5-Dimethylphenyl-oxymethyl)phenyl]-3-methoxy-acrylsäuremethylester, 2-(2-(3-(2,6-dichlorphenyl)-1-methyl-allylideneaminooxymethyl)-phenyl)-2-methoxyimino-N-methyl-acetamide;
B) Carbonsäureamide:
   - Carbonsäureanilide: Benalaxyl, Benalaxyl-M, Benodanil, Bixafen, Boscalid, Carboxin, Fenfuram, Fenhexamid, Flutolanil, Furametpyr, Isopyrazam, Isotianil, Kiralaxyl, Mepronil, Metalaxyl, Metalaxyl-M (Mefenoxam), Ofurace, Oxadixyl, Oxycarboxin, Penflufen (N-(2-(1,3-Dimethyl-butyl)-phenyl)-1,3-dimethyl-5-fluor-1 H-pyrazol-4-carboxamid), Penthiopyrad, Sedaxane, Tecloftalam, Thifluzamide, Tiadinil, 2-Amino-4-methyl-thiazol-5-carboxanilid, N-(3',4',5'-Trifluorbiphenyl-2-yl)-3-difluormethyl-1-methyl-1 H-pyrazol-4-carboxamid, N-(4'-Trifluormethylthiobiphenyl-2-yl)-3-difluormethyl-1-methyl-1 H-pyrazol-4-carboxamid, N-(2-(1,3,3-Trimethyl-butyl)-phenyl)-1,3-dimethyl-5-fluor-1 H-pyrazol-4-carboxamid;
   - Carbonsäuremorpholide: Dimethomorph, Flumorph, Pyrimorph;
   - Benzoesäureamide: Flumetover, Fluopicolide, Fluopyram, Zoxamid;
   - Sonstige Carbonsäureamide: Carpropamid, Diclocymet, Mandipropamid, Oxytetracyclin, Silthiofam, N-(6-methoxy-pyridin-3-yl)cyclopropancarbonsäureamid;
C) Azole:
   - Triazole: Azaconazol, Bitertanol, Bromuconazol, Cyproconazol, Difenoconazol, Diniconazol, Diniconazol-M, Epoxiconazol, Fenbuconazol, Fluquinconazol, Flusilazol, Flutriafol, Hexaconazol, Imibenconazol, Ipconazol, Metconazol, Myclobutanil, Oxpoconazol, Paclobutrazol, Penconazol, Propiconazol, Prothioconazol, Simeconazol, Tebuconazol, Tetraconazol, Triadimefon, Triadimenol, Triticonazol, Uniconazol;
   - Imidazole: Cyazofamid, Imazalil, Imazalilsulfat, Pefurazoat, Prochloraz, Triflumizol;
   - Benzimidazole: Benomyl, Carbendazim, Fuberidazole, Thiabendazol;
   - Sonstige: Ethaboxam, Etridiazol, Hymexazol, 2-(4-Chlor-phenyl)-N-[4-(3,4-dimethoxy-phenyl)-isoxazol-5-yl]-2-prop-2-inyloxy-acetamid;
D) Stickstoffhaltige Heterocyclylverbindungen
   - Pyridine: Fluazinam, Pyrifenox, 3-[5-(4-Chlor-phenyl)-2,3-dimethyl-isoxazolidin-3-yl]-pyridin, 3-[5-(4-Methyl-phenyl)-2,3-dimethyl-isoxazolidin-3-yl]-pyridin;
   - Pyrimidine: Bupirimat, Cyprodinil, Diflumetorim, Fenarimol, Ferimzone, Mepanipyrim, Nitrapyrin, Nuarimol, Pyrimethanil;
   - Piperazine: Triforine;
   - Pyrrole: Fludioxonil, Fenpiclonil;
   - Morpholine: Aldimorph, Dodemorph, Dodemorphacetat, Fenpropimorph, Tridemorph;
   - Piperidine: Fenpropidin;
   - Dicarboximide: Fluorimid, Iprodione, Procymidone, Vinclozolin;
   - nichtaromatische 5-Ring-Heterocyclen: Famoxadon, Fenamidon, Flutianil, Octhilinon, Probenazol, 5-Amino-2-isopropyl-3-oxo-4-ortho-tolyl-2,3-dihydropyrazol-1-thiocarbonsäureS-allylester;
   - sonstige: Acibenzolar-S-methyl, Amisulbrom, Anilazin, Blasticidin-S, Captafol, Captan, Chinomethionat, Dazomet, Debacarb, Diclomezine, Difenzoquat, Difenzoquatmethylsulfat, Fenoxanil, Folpet, Oxolinsäure, Piperalin, Proquinazid, Pyroquilon, Quinoxyfen, Triazoxid, Tricyclazol, 2-Butoxy-6-jod-3-propyl-chromen-4-on, 5-Chlor-1-(4,6-dimethoxy-pyrimidin-2-yl)-2-methyl-1 H-benzoimidazol, 5-Chlor-7-(4-methylpiperidin-1-yl)-6-(2,4,6-trifluor-phenyl)-[1,2,4]triazolo[1,5-a]pyrimidin, 5-Ethyl-6-octyl-[1,2,4]triazolo[1,5-a]pyrimidin-7-ylamin;
E) Carbamate und Dithiocarbamate
   - Thio- und Dithiocarbamate: Ferbam, Mancozeb, Maneb, Metam, Methasulphocarb, Metiram, Propineb, Thiram, Zineb, Ziram;
   - Carbamate: Diethofencarb, Benthiavalicarb, Iprovalicarb, Propamocarb, Propamocarb-hydrochlorid, Valiphenal, N-(1-(1-(4-Cyanophenyl)ethansulfonyl)-but-2-yl)carbaminsäure-(4-fluorphenyl)ester;
F) Sonstige Fungizide
   - Guanidine: Dodine, Dodine freie Base, Guazatin, Guazatinacetat, Iminoctadin, Iminoctadin-triacetat, Iminoctadin-tris(albesilat);
   - Antibiotika: Kasugamycin, Kasugamycinhydrochlorid-Hydrat, Polyoxine, Streptomycin, Validamycin A;
   - Nitrophenylderivate: Binapacryl, Dicloran, Dinobuton, Dinocap, Nitrothal-isopropyl, Tecnazen;
   - Organometallverbindungen: Fentin-Salze wie beispielsweise Fentin-acetat, Fentinchlorid, Fentin-hydroxid;
   - Schwefelhaltige Heterocyclylverbindungen: Dithianon, Isoprothiolane;
   - Organophosphorverbindungen: Edifenphos, Fosetyl, Fosetyl-Aluminium, Iprobenfos, Phosphorige Säure und ihre Salze, Pyrazophos, Tolclofos-methyl;
   - Organochlorverbindungen: Chlorthalonil, Dichlofluanid, Dichlorphen, Flusulfamide, Hexachlorbenzol, Pencycuron, Pentachlorphenol und dessen Salze, Phthalid, Quintozen, Thiophanat-Methyl, Tolylfluanid, N-(4-Chlor-2-nitro-phenyl)-N-ethyl-4-methyl-benzolsulfonamid;
   - Anorganische Wirkstoffe: Phosphorige Säure und ihre Salze, Bordeaux Brühe, Kupfersalze wie beispielsweise Kupferacetat, Kupferhydroxid, Kupferoxychlorid, basisches Kupfersulfat, Schwefel;
   - Biologische Pilzbekämpfungsmittel, Pflanzenstärkungsmittel: *Bacillus subtilis*-Stamm NRRL-Nr. B-21661 (z.B. die Produkte RHAPSODY^{®}, SERENADE^{®} MAX und SERENADE^{®} ASO der Fa. AgraQuest, Inc., USA.), *Bacillus pumilus-Stamm* NRRL-Nr. B-30087 (z.B. SONATA^{®} and BALLAD^{®} Plus der Fa. AgraQuest, Inc., USA), *Ulocladium oudemansii* (z.B. BOTRY-ZEN der Fa. BotriZen Ltd., Neuseeland), Chitosan (z.B. ARMOUR-ZEN der Fa. BotriZen Ltd., Neuseeland).
   - Sonstige: Biphenyl, Bronopol, Cyflufenamid, Cymoxanil, Diphenylamin, Metrafenon, Mildiomycin, Oxin-Kupfer, Prohexadion-Calcium, Spiroxamin, Tolylfluanid, N-(Cyclopropylmethoxyimino-(6-difluormethoxy-2,3-difluor-phenyl)-methyl)-2-phenylacetamid, N'-(4-(4-Chlor-3-trifluormethyl-phenoxy)-2,5-dimethyl-phenyl)-N-ethyl-N-methylformamidin, N'-(4-(4-Fluor-3-trifluormethyl-phenoxy)-2,5-dimethyl-phenyl)-N-ethyl-N-methylformamidin, N'-(2-Methyl-5-trifluormethyl-4-(3-trimethylsilanyl-propoxy)-phenyl)-N-ethyl-N-methylformamidin, N'-(5-Difluormethyl-2-methyl-4-(3-trimethylsilanylpropoxy)-phenyl)-N-ethyl-N-methylformamidin, 2-{1-[2-(5-Methyl-3-trifluormethylpyrazol-1-yl)-acetyl]-piperidin-4-yl}-thiazol-4-carboxylsäure-methyl-(1,2,3,4-tetrahydronaphthalen-1-yl)-amid, 2-{1-[2-(5-Methyl-3-trifluormethyl-pyrazol-1-yl)-acetyl]-piperidin-4-yl}-thiazol-4-carboxylsäure-methyl-(R)-1,2,3,4-tetrahydronaphthalen-1-yl-amid, Essigsäure-6-tert.-butyl-8-fluor-2,3-dimethyl-quinolin-4-yl-ester, Methoxy-essigsäure-6-tert.-butyl-8-fluor-2,3-dimethyl-quinolin-4-yl-ester, *N*-Methyl-2-{1-[2-(5-methyl-3-trifluormethyl-1H-pyrazol-1-yl)-acetyl]-piperidin-4-yl}-N[(1R)-1,2,3,4-tetrahydronaphthalen-1-yl]-4-thiazolcarboxamid;
G) Wachstumsregler
   Abscisinsäure, Amidochlor, Ancymidol, 6-Benzylaminopurin, Brassinolid, Butralin, Chlormequat (Chlormequatchlorid), Cholinchlorid, Cyclanilid, Daminozid, Dikegulac, Dimethipin, 2,6-Dimethylpuridin, Ethephon, Flumetralin, Flurprimidol , Fluthiacet, Forchlorfenuron, Gibberellinsäure, Inabenfid, Indol-3-essigsäure, Maleinsäurehydrazid, Mefluidid, Mepiquat (Mepiquatchlorid), Metconazol, Naphthalenessigsäure, N-6-Benzyladenin, Paclobutrazol, Prohexadion (Prohexadion-Calcium), Prohydrojasmon, Thidiazuron, Triapenthenol, Tributylphosphorotrithioat, 2,3,5-tri-Jodbenzoesäure, Trinexapac-ethyl und Uniconazol;
H) Herbizide
   - Acetamide: Acetochlor, Alachlor, Butachlor, Dimethachlor, Dimethenamid, Flufenacet, Mefenacet, Metolachlor, Metazachlor, Napropamid, Naproanilid, Pethoxamid, Pretilachlor, Propachlor, Thenylchlor;
   - Aminosäureanaloga: Bilanafos, Glyphosat, Glufosinat, Sulfosat;
   - Aryloxyphenoxypropionate: Clodinafop, Cyhalofop-butyl, Fenoxaprop, Fluazifop, Haloxyfop, Metamifop, Propaquizafop, Quizalofop, Quizalofop-P-tefuryl;
   - Bipyridyle: Diquat, Paraquat;
   - Carbamate und Thiocarbamate: Asulam, Butylate, Carbetamide, Desmedipham, Dimepiperat, Eptam (EPTC), Esprocarb, Molinate, Orbencarb, Phenmedipham, Prosulfocarb, Pyributicarb, Thiobencarb, Triallate;
   - Cyclohexanedione: Butroxydim, Clethodim, Cycloxydim, Profoxydim, Sethoxydim, Tepraloxydim, Tralkoxydim;
   - Dinitroaniline: Benfluralin, Ethalfluralin, Oryzalin, Pendimethalin, Prodiamine, Trifluralin;
   - Diphenylether: Acifluorfen, Aclonifen, Bifenox, Diclofop, Ethoxyfen, Fomesafen, Lactofen, Oxyfluorfen;
   - Hydroxybenzonitrile: Bromoxynil, Dichlobenil, loxynil;
   - Imidazolinone: Imazamethabenz, Imazamox, Imazapic, Imazapyr, Imazaquin, Imazethapyr;
   - Phenoxyessigsäuren: Clomeprop, 2,4-Dichlorphenoxyessigsäure (2,4-D), 2,4-DB, Dichlorprop, MCPA, MCPA-thioethyl, MCPB, Mecoprop;
   - Pyrazine: Chloridazon, Flufenpyr-ethyl, Fluthiacet, Norflurazon, Pyridat;
   - Pyridine: Aminopyralid, Clopyralid, Diflufenican, Dithiopyr, Fluridone, Fluroxypyr, Picloram, Picolinafen, Thiazopyr;
   - Sulfonylharnstoffe: Amidosulfuron, Azimsulfuron, Bensulfuron, Chlorimuron-Ethyl, Chlorsulfuron, Cinosulfuron, Cyclosulfamuron, Ethoxysulfuron, Flazasulfuron, Flucetosulfuron, Flupyrsulfuron, Foramsulfuron, Halosulfuron, Imazosulfuron, lodosulfuron, Mesosulfuron, Metsulfuron-methyl, Nicosulfuron, Oxasulfuron, Primisulfuron, Prosulfuron, Pyrazosulfuron, Rimsulfuron, Sulfometuron, Sulfosulfuron, Thifensulfuron, Triasulfuron, Tribenuron, Trifloxysulfuron, Triflusulfuron, Tritosulfuron, 1-((2-Chlor-6-propyl-imidazo[1,2-b]pyridazin-3-yl)sulfonyl)-3-(4,6-dimethoxy-pyrimidin-2-yl)harnstoff;
   - Triazine: Ametryn, Atrazin, Cyanazin, Dimethametryn, Ethiozin, Hexazinon, Metamitron, Metribuzin, Prometryn, Simazin, Terbuthylazin, Terbutryn, Triaziflam;
   - Harnstoffe: Chlorotoluron, Daimuron, Diuron, Fluometuron, Isoproturon, Linuron, Methabenzthiazuron,Tebuthiuron;
   - andere Hemmstoffe der Acetolactatsynthase: Bispyribac-Natrium, Cloransulam-Methyl, Diclosulam, Florasulam, Flucarbazone, Flumetsulam, Metosulam, Orthosulfamuron, Penoxsulam, Propoxycarbazone, Pyribambenz-Propyl, Pyribenzoxim, Pyriftalid, Pyriminobac-methyl, Pyrimisulfan, Pyrithiobac, Pyroxasulfon, Pyroxsulam;
   - Sonstige: Amicarbazon, Aminotriazol, Anilofos, Beflubutamid, Benazolin, Bencarbazon, Benfluresat, Benzofenap, Bentazon, Benzobicyclon, Bromacil, Bromobutid, Butafenacil, Butamifos, Cafenstrole, Carfentrazone, Cinidon-Ethlyl, Chlorthal, Cinmethylin, Clomazone, Cumyluron, Cyprosulfamid, Dicamba, Difenzoquat, Diflufenzopyr, *Drechsiera monoceras,* Endothal, Ethofumesat, Etobenzanid, Fentrazamide, Flumiclorac-Pentyl, Flumioxazin, Flupoxam, Fluorochloridon, Flurtamon, Indanofan, Isoxaben, Isoxaflutol, Lenacil, Propanil, Propyzamid, Quinclorac, Quinmerac, Mesotrion, Methylarsensäure, Naptalam, Oxadiargyl, Oxadiazon, Oxaziclomefon, Pentoxazon, Pinoxaden, Pyraclonil, Pyraflufen-Ethyl, Pyrasulfotol, Pyrazoxyfen, Pyrazolynat, Quinoclamin, Saflufenacil, Sulcotrion, Sulfentrazon, Terbacil, Tefuryltrion, Tembotrion, Thiencarbazon, Topramezon, 4-Hydroxy-3-[2-(2-methoxy-ethoxymethyl)-6-trifluormethyl-pyridin-3-carbonyl]-bicyclo[3.2.1]oct-3-en-2-on, (3-[2-Chlor-4-fluor-5-(3-methyl-2,6-dioxo-4-trifluormethyl-3,6-dihydro-2H-pyrimidin-1-yl)-phenoxy]-pyridin-2-yloxy)-essigsäureethylester, 6-Amino-5-chlor-2-cyclopropylpyrimidin-4-carboxylsäuremethylester, 6-Chlor-3-(2-cyclopropyl-6-methyl-phenoxy)-pyridazin-4-ol, 4-Amino-3-chlor-6-(4-chlor-phenyl)-5-fluor-pyridin-2-carboxylsäure, 4-Amino-3-chlor-6-(4-chlor-2-fluor-3-methoxy-phenyl)-pyridin-2-carboxylsäuremethylester und 4-Amino-3-chlor-6-(4-chloro-3-dimethylamino-2-fluor-phenyl)-pyridin-2-carboxylsäuremethylester;
I) Insektizide
   - Organo(thio)phosphate: Acephat, Azamethiphos, Azinphos-methyl, Chlorpyrifos, Chlorpyrifos-Methyl, Chlorfenvinphos, Diazinon, Dichlorvos, Dicrotophos, Dimethoat, Disulfoton, Ethion, Fenitrothion, Fenthion, Isoxathion, Malathion, Methamidophos, Methidathion, Methyl-Parathion, Mevinphos, Monocrotophos, Oxydemeton-Methyl, Paraoxon, Parathion, Phenthoate, Phosalone, Phosmet, Phosphamidon, Phorate, Phoxim, Pirimiphos-Methyl, Profenofos, Prothiofos, Sulprophos, Tetrachlorvinphos, Terbufos, Triazophos, Trichlorfon;
   - Carbamate: Alanycarb, Aldicarb, Bendiocarb, Benfuracarb, Carbaryl, Carbofuran, Carbosulfan, Fenoxycarb, Furathiocarb, Methiocarb, Methomyl, Oxamyl, Pirimicarb, Propoxur, Thiodicarb, Triazamate;
   - Pyrethroide: Allethrin, Bifenthrin, Cyfluthrin, Cyhalothrin, Cyphenothrin, Cypermethrin, alpha-Cypermethrin, beta-Cypermethrin, zeta-Cypermethrin, Deltamethrin, Esfenvalerat, Etofenprox, Fenpropathrin, Fenvalerate, Imiprothrin, Lambda-Cyhalothrin, Permethrin, Prallethrin, Pyrethrin I und II, Resmethrin, Silafluofen, tau-Fluvalinat, Tefluthrin, Tetramethrin, Tralomethrin, Transfluthrin, Profluthrin, Dimefluthrin,
   - Hemmstoffe des Insektenwachstums: a) Chitinsynthese-Hemmstoffe: Benzoylharnstoffe: Chlorfluazuron, Cyramazin, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazol, Clofentazin; b) Ecdyson-Antagonisten: Halofenozid, Methoxyfenozid, Tebufenozid, Azadirachtin; c) Juvenoide: Pyriproxyfen, Methoprene, Fenoxycarb; d) Lipidbiosynthese-Hemmstoffe: Spirodiclofen, Spiromesifen, Spirotetramat;
   - Nikotinreceptor-Agonisten/Antagonisten: Clothianidin, Dinotefuran, Imidacloprid, Thiamethoxam, Nitenpyram, Acetamiprid, Thiacloprid, 1-(2-chloro-thiazol-5-yl-methyl)-2-nitrimino-3,5-dimethyl-[1,3,5]triazinan;
   - GABA-Antagonisten: Endosulfan, Ethiprol, Fipronil, Vaniliprol, Pyrafluprol, Pyriprol, 5-Amino-1-(2,6-dichlor-4-methyl-phenyl)-4-sulfinamoyl-1 H-pyrazol-3-thiocarbonsäureamid;
   - Macrocyclische Lactone: Abamectin, Emamectin, Milbemectin, Lepimectin, Spinosad, Spinetoram;
   - Mitochondriale Elektronentransportketten-Inhibitor (METI) I Akarizide: Fenazaquin, Pyridaben, Tebufenpyrad, Tolfenpyrad, Flufenerim;
   - METI II und III Substanzen: Acequinocyl, Fluacyprim, Hydramethylnon;
   - Entkoppler: Chlorfenapyr;
   - Hemmstoffe der oxidativen Phosphorylierung: Cyhexatin, Diafenthiuron, Fenbutatinoxid, Propargit;
   - Hemmstoffe der Häutung der Insekten: Cryomazin;
   - Hemmstoffe von ,mixed function oxidases': Piperonylbutoxid;
   - Natriumkanalblocker: Indoxacarb, Metaflumizon;
   - Sonstige: Benclothiaz, Bifenazate, Cartap, Flonicamid, Pyridalyl, Pymetrozin, Schwefel, Thiocyclam, Flubendiamid, Chlorantraniliprol, Cyazypyr (HGW86); Cyenopyrafen, Flupyrazofos, Cyflumetofen, Amidoflumet, Imicyafos, Bistrifluron und Pyrifluquinazon.

Bevorzugt Pestizide umfassen mindestens ein Pestizid mit mindestens einer H-aciden Gruppe (wie Carbonsäuregruppe, Phosphonsäuregruppe, Phosphinsäuregruppe) oder deren anionische Salze (z. B. Mono-, Di-, oder Trisalze). Diese anionischen Salze der Pestizide mit einer H-aciden Gruppe sind auch als anionische Pestizide in der Gruppe A⁻ geeignet. Bevorzugte Pestizide mit einer H-aciden Gruppe sind Herbizide mit einer H-aciden Gruppe. Beispiele für Herbizide mit einer H-aciden Gruppen sind Aminosäureanaloga (wie Glyphosate oder Glufosinate) oder Imidazolinone (wie Imazamethabenz, Imazamox, Imazapic, Imazapyr, Imazaquin, Imazethapyr).
Besonders bevorzugte Pestizide mit einer H-aciden gruppen sind Glyphosat und Glufosinat. In einer weiteren bevorzugten Ausführungsform sind Pestizide mit einer H-aciden Gruppe Imidazolinone.

Besonders bevorzugt umfasst das Pestizid ein Pestizid mit einer H-aciden Gruppe und ein weiteres Pestizid. In einer weiteren Ausführungsform umfasst das Pestizid Mischungen von mindestens zwei Pestiziden mit einer H-aciden Gruppe, und optional weitere Pestizide (wie mindestens ein Fungizid, Herbizid, Insektizid, und/oder Safener, wobei Fungizide und/oder Herbizide bevorzugt sind).

In einer weiteren bevorzugten Ausführungsform umfasst das Pestizide Glyphosat (z.B. als freie Säure, Natriumsalz, Sesquinatriumsalz, Kaliumsalz, Dikaliumsalz, Ammoniumsalz, Diammoniumsalz, Dimethylammoniumsalz, Trimesiumsalz oder Isopropylaminsalz) oder Glufosinat (z.B. als Ammoniumsalz). Besonders bevorzugt umfasst das Pestizid Glyphosat (z.B. als Kaliumsalz, Ammoniumsalz, Isopropylaminsalz). Besonders bevorzugt umfasst das Pestizid Glyphosat oder Glufosinat, und zusätzlich ein weiteres Herbizid. In einer weiteren bevorzugten Ausführungsform umfasst das Pestizid Glyphosat oder Glufosinat, und zusätzlich ein weiteres Pestizid (wie mindestens ein Fungizid, Herbizid, Insektizid, und/oder Safener, wobei Fungizide und/oder Herbizide bevorzugt sind).

Die erfindungsgemäßen Zusammensetzungen können weiterhin auch für agrochemische Formulierungen übliche Hilfsmittel enthalten, wobei sich die Wahl der Hilfsmittel nach der konkreten Anwendungsform, dem Formulierungstyp bzw. dem Wirkstoff richtet. Beispiele für geeignete Hilfsmittel sind Lösungsmittel, feste Trägerstoffe, oberflächenaktive Stoffe (wie Tenside, Solubilisatoren, Schutzkolloide, Netzmittel und Haftmittel), organische und anorganische Verdicker, Bakterizide, Frostschutzmittel, Entschäumer, ggf. Farbstoffe und Kleber (z. B. für Saatgutbehandlung) oder übliche Hilfsmittel für Köderformulierungen (z. B. Lockstoffe, Futtermittel, Bitterstoffe).

Als Lösungsmittel kommen Wasser oder organische Lösungsmittel wie Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Gykole, Ketone wie Cyclohexanon, gamma-Butyrolacton, Dimethylfettsäureamide, Fettsäuren und Fettsäureester und stark polare Lösungsmittel, z.B. Amine wie N-Methylpyrrolidon, in Betracht. Grundsätzlich können auch Lösungsmittelgemische verwendet werden sowie Gemische aus den vorstehend genannten Lösungsmitteln und Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Als oberflächenaktive Stoffe (Adjuvantien, Netz-, Haft-, Dispergier- oder Emulgiermittel) kommen die Alkali-, Erdalkali-, Ammoniumsalze von aromatischen Sulfonsäuren, z. B. von Lignin-(Borresperse^{®}-Typen, Borregaard, Norwegen), Phenol-, Naphthalin-(Morwet^{®}-Typen, Akzo Nobel, USA) und Dibutylnaphthalinsulfonsäure (Nekal^{®}-Typen, BASF, Deutschland), sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolethern, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol^{®}-Typen, Clariant, Schweiz), Polycarboxylate (Sokalan^{®}-Typen, BASF, Deutschland), Polyalkoxylate, Polyvinylamin (Lupamin^{®}-Typen, BASF, Deutschland), Polyethylenimin (Lupasol^{®}-Typen, BASF, Deutschland), Polyvinylpyrrolidon und deren Copolymere in Betracht.

Die erfindungsgemäße Zusammensetzung kann 0,1 bis 40 Gew.%, bevorzugt 1 bis 30 und insbesondere 2 bis 20 Gew.% oberflächenaktive Stoffe umfassen, wobei die Menge des Alkoxlylats (A) und (AQ) nicht mit einberechnet werden.

Geeignete Verdicker sind Verbindungen, die der Formulierung ein modifiziertes Fließverhalten verleihen, d. h. eine hohe Viskosität im Ruhezustand und niedrige Viskosität im bewegten Zustand. Beispiele sind Polysaccharide, Proteine (wie Casein oder gelatine), synthetische Polymere, oder anorganische Schichtmineralien. Solche Verdicker sind kommerziell erhältlich, beispielsweise Xanthan Gum (Kelzan^{®}, CP Kelco, USA), Rhodopol^{®} 23 (Rhodia, Frankreich) oder Veegum^{®} (R.T. Vanderbilt, USA) oder Attaclay^{®} (Engelhard Corp., NJ, USA). Der Gehalt an Verdicker in der Formulierung richtet sich nach der Wirksamkeit des Verdickers. Der Fachmann wird einen Gehalt wählen, um die gewünschte Viskosität der Formulierung zu erhalten. Der Gehalt wird meist 0,01 bis 10 Gew.% betragen.

Bakterizide können zur Stabilisierung der Zusammensetzung zugesetzt werden. Beispiele für Bakterizide sind solche basierend auf Diclorophen und Benzylalkoholhemiformal sowie Isothiazolinonderivaten wie Alkylisothiazolinonen und Benzisothiazolinonen (Acticide^{®} MBS der Fa. Thor Chemie). Beispiele für geeignete Frostschutzmittel sind Ethylenglycol, Propylenglycol, Harnstoff und Glycerin. Beispiele für Entschäumer sind Silikonemulsionen (wie z. B. Silikon^{®} SRE, Wacker, Deutschland oder Rhodorsil^{®}, Rhodia, Frankreich), langkettige Alkohole, Fettsäuren, Salze von Fettsäuren, fluororganische Verbindungen und deren Gemische.

Die erfindungsgemäße Zusammensetzung kann bevorzugt in Form einer agroschemischen Formulierung vorliegen. Beispiele für solche Formulierungen und ihrer Herstellung sind:
i) Wasserlösliche Konzentrate (SL, LS): 10 Gew.-Teile der Wirkstoffe werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Zusammensetzung mit 10 Gew.-% Wirkstoffgehalt.
ii) Dispergierbare Konzentrate (DC): 20 Gew.-Teile der Wirkstoffe werden in 70 Gew.-Teilen Cyclohexanon unter Zusatz von 10 Gew.-Teilen eines Dispergiermittels z. B. Polyvinylpyrrolidon gelöst. Bei Verdünnung in Wasser ergibt sich eine Dispersion. Der Wirkstoffgehalt beträgt 20 Gew.-%
iii) Emulgierbare Konzentrate (EC): 15 Gew.-Teile der Wirkstoffe werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Zusammensetzung hat 15 Gew.-% Wirkstoffgehalt.
iv) Emulsionen (EW, EO, ES): 25 Gew.-Teile der Wirkstoffe werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z. B. Ultra-Turrax) in 30 Gew.-Teile Wasser gegeben und zu einer homogenen Emulsion gebracht. Bei der Verdünnung in Wasser ergibt sich eine Emulsion. Die Zusammensetzung hat einen Wirkstoffgehalt von 25 Gew.-%.
v) Suspensionen (SC, OD, FS): 20 Gew.-Teile der Wirkstoffe werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Zusammensetzung beträgt 20 Gew.-%.
vi) Wasserdispergierbare und wasserlösliche Granulate (WG, SG): 50 Gew.-Teile der Wirkstoffe werden unter Zusatz von 50 Gew.-Teilen Dispergier- und Netzmitteln fein gemahlen und mittels technischer Geräte (z. B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Zusammensetzung hat einen Wirkstoffgehalt von 50 Gew.-%.
vii) Wasserdispergierbare und wasserlösliche Pulver (WP, SP, SS, WS): 75 Gew.-Teile der Wirkstoffe werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Zusammensetzung beträgt 75 Gew.-%.
viii) Gele (GF): In einer Kugelmühle werden 20 Gew.-Teile der Wirkstoffe, 10 Gew.-Teile Dispergiermittel, 1 Gew.-Teil Quellmittel ("gelling agent") und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.
ix) Stäube (DP, DS): 5 Gew.-Teile der Wirkstoffe werden fein gemahlen und mit 95 Gew.-Teilen feinteiligem Kaolin innig vermischt. Man erhält dadurch ein Stäubemittel mit 5 Gew.-% Wirkstoffgehalt.
x) Granulate (GR, FG, GG, MG): 0,5 Gew.-Teile der Wirkstoffe werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.
xi) ULV- Lösungen (UL): 10 Gew.-Teile der Wirkstoffe werden in 90 Gew.-Teilen eines organischen Lösungsmittel z. B. Xylol gelöst. Dadurch erhält man eine Zusammensetzung für die Direktapplikation mit 10 Gew.-% Wirkstoffgehalt.

Die Zusammensetzungen enthalten im allgemeinen 0,01 bis 95 Gew.-%, vorzugsweise 0,1 bis 90 Gew.-% der Pestizide.

Der Anwender verwendet die erfindungsgemäße Zusammensetzung üblicherweise für die Anwendung in einer Vordosiereinrichtung, im Rückenspritzer, im Spritztank oder im Sprühflugzeug. Dabei wird die Formulierung mit Wasser und/oder Puffer auf die gewünschte Anwendungskonzentration gebracht, wobei gegebenenfalls weitere Hilfsstoffe zugegeben werden, und so die anwendungsbereite Spritzbrühe (sog. Tankmix) erhalten wird. Üblicherweise werden 50 bis 500 Liter der anwendungsbereiten Spritzbrühe pro Hektar landwirtschaftlicher Nutzfläche aufgebracht, bevorzugt 100 bis 400 Liter. In speziellen Segmenten können diese Mengen auch über- (z.B. Obstbau) oder unterschritten (z.B. Flugzeugapplikation) werden. Die Wirkstoffkonzentrationen in den anwendungsfertigen Zubereitungen können in größeren Bereichen variiert werden. Im Allgemeinen liegen sie zwischen 0,0001 und 10%, vorzugsweise zwischen 0,01 und 1%.

Zu den Wirkstoffen oder den diese enhaltenden Zusammensetzungen können Öle verschiedenen Typs, Netzmittel, Driftreduktionsmittel, Sticker, Spreader, Adjuvantien, Dünger, Pflanzenstärkungsmittel, Spurenelemente, Herbizide, Bakterizide, Fungizide und/oder Schädlingsbekämpfungsmittel, gegebenenfalls auch erst unmittelbar vor der Anwendung, dem Tankmix zugesetzt werden. Diese Mittel können zu den erfindungsgemäßen Zusammensetzungen im Gewichtsverhältnis 1:100 bis 100:1, bevorzugt 1:10 bis 10:1 zugemischt werden. Als Adjuvanzien in diesem Sinne kommen insbesondere in Frage: organisch modifizierte Polysiloxane, z. B. Break Thru S 240^{®}; Alkoholalkoxylate, z. B. Atplus^{®} 245, Atplus^{®} MBA 1303, Plurafac^{®} LF 300 und Lutensol^{®} ON 30; EO-PO-Blockpolymerisate, z. B. Pluronic^{®} RPE 2035 und Genapol^{®} B; Alkoholethoxylate, z. B. Lutensol^{®} XP 80; und Natriumdioctylsulfosuccinat, z. B. Leophen^{®} RA.

Die Aufwandmengen des Wirkstoffs liegen bei der Anwendung im Pflanzenschutz je nach Art des gewünschten Effektes zwischen 0,001 und 2,0 kg Wirkstoff pro ha, bevorzugt zwischen 0,005 und 2 kg pro ha, besonders bevorzugt zwischen 0,05 und 0,9 kg pro ha, insbesondere zwischen 0,1 und 0,75 kg pro ha.

Die vorliegende Erfindung betrifft weiterhin ein Verfahren zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die erfindungsgemäße Zusammensetzung auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt.

Geeignete Nutzplanzen sind beispielsweise Getreide, z. B. Weizen, Roggen, Gerste, Triticale, Hafer oder Reis; Rüben, z. B. Zucker oder Futterrüben; Kern-, Stein und Beerenobst, z. B. Äpfel, Birnen, Pflaumen, Pfirsiche, Mandeln, Kirschen, Erdbeeren, Himbeeren, Johannisbeeren oder Stachelbeeren; Leguminosen, z. B. Bohnen, Linsen, Erbsen, Luzerne oder Soja; Ölpflanzen, z. B. Raps, Senf, Oliven, Sonnenblumen, Kokosnuss, Kakao, Rizinusbohnen, Ölpalme, Erdnüsse oder Soja; Kürbisgewächse, z. B. Kürbissse, Gurken oder Melonen; Faserpflanzen, z. B. Baumwolle, Flachs, Hanf oder Jute; Zitrusfrüchte, z. B. Orangen, Zitronen, Pampelmusen oder Mandarinen; Gemüsepflanzen, z. B. Spinat, Salat, Spargel, Kohlpflanzen, Möhren, Zwiebeln, Tomaten, Kartoffeln, Kürbis oder Paprika; Lorbeergewächse, z. B. Avocados, Zimt oder Kampher; Energie- und Rohstoffpflanzen, z. B. Mais, Soja, Weizen, Raps, Zuckerrohr oder Ölpalme; Mais; Tabak; Nüsse; Kaffee; Tee; Bananen; Wein (Tafel- und Weintrauben); Hopfen; Gras, z. B. Rasen; Süßkraut (*Stevia rebaudania*); Kautschukpflanz - und Forstpflanzen, z. B. Blumen, Sträucher, Laub- und Nadelbäume sowie Vermehrungsmaterial, z. B. Samen, und Erntegut dieser Pflanzen.

Der Begriff Nutzplanzen schließt auch solche Pflanzen ein, die durch Züchtung, Mutagenese oder gentechnische Methoden verändert wurden einschließlich der auf dem Markt oder in Entwicklung befindlichen biotechnologischen Agrarprodukte. Gentechnisch veränderte Pflanzen sind Pflanzen, deren genetisches Material in einer Weise verändert worden ist, wie sie unter natürlichen Bedingungen durch Kreuzen, Mutationen oder natürliche Rekombination (d.h. Neuzusammenstellung der Erbinformation) nicht vorkommt. Dabei werden in der Regel ein oder mehrere Gene in das Erbgut der Pflanze integriert, um die Eigenschaften der Pflanze zu verbessern. Derartige gentechnische Veränderungen umfassen auch posttranslationale Modifikationen von Proteinen, Oligo- oder Polypeptiden z.B. mittels Glykosylierung oder Bindung von Polymeren wie z.B. prenylierter, acetylierter oder farneylsierter Reste oder PEG-Reste.

Beispielhaft seien Pflanzen genannt, die durch züchterische und gentechnische Maßnahmen eine Toleranz gegen bestimmter Herbizidklassen, wie Hydroxyphenylpyruvat-Dioxygenase (HPPD)-Inhibitoren, Acetolactat-Synthase (ALS)-Inhibitoren wie z. B. Sulfonylharnstoffe (EP-A 257 993, US 5,013,659) oder Imidazolinone (z. B. US 6,222,100, WO 01/82685, WO 00/26390, WO 97/41218, WO 98/02526, WO 98/02527,

WO 04/106529, WO 05/20673, WO 03/14357, WO 03/13225, WO 03/14356,
WO 04/16073), Enolpyruvylshikimat-3-Phosphat-Synthase (EPSPS)-Inhibitoren wie z. B. Glyphosat (siehe z. B. WO 92/00377), Glutaminsynthetase (GS)-Inhibitoren wie z. B. Glufosinat (siehe z. B. EP-A 242 236, EP-A 242 246) oder Oxynil-Herbizide (siehe z. B. US 5,559,024) erworben haben. Durch Züchtung und Mutagenese wurde z. B. Clearfield^{®}-Raps (BASF SE, Deutschland) erzeugt, der eine Toleranz gegen Imidazolinone, z. B. Imazamox, hat. Mit Hilfe gentechnischer Methoden wurden Kulturpflanzen, wie Soja, Baumwolle, Mais, Rüben und Raps, erzeugt, die resistent gegen Glyphosat oder Glufosinat sind, erzeugt, welche unter den Handelsnamen RoudupReady^{®} (Glyphosat-resistent, Monsanto, U.S.A.) und Liberty Link^{®} (Glufosinat-resistent, Bayer CropScience, Deutschland) erhältlich sind.

Weiterhin sind auch Pflanzen umfasst, die mit Hilfe gentechnischer Maßnahmen ein oder mehrere Toxine, z. B. solche aus dem Bakterienstamm *Bacillus*, produzieren. Toxine, die durch solche gentechnisch veränderten Pflanzen hergestellt werden, umfassen z. B. insektizide Proteine von *Bacillus* spp., insbesondere von *B*. *thuringiensis,* wie die Endotoxine Cry1Ab, Cry1Ac, Cry1F, Cry1 Fa2, Cry2Ab, Cry3A, Cry3Bb1, Cry9c, Cry34Ab1 oder Cry35Ab1; oder vegetative insektizide Proteine (VIPs), z. B. VIP1, VIP2, VIP3, oder VIP3A; insektizide Proteine von Nematoden-kolonisierenden Bakterien, z. B. *Photorhabdus* spp. oder *Xenorhabdus* spp.; Toxine aus tierischen Organismen, z. B. Wepsen,-, Spinnen- oder Skorpionstoxine; pilzliche Toxine, z. B. aus Streptomyceten; pflanzliche Lektine, z. B. aus Erbse oder Gerste; Agglutinine; Proteinase-Inhibitoren, z. B. Trypsin-Inhibitoren, Serinprotease-Inhibitoren, Patatin, Cystatin oder Papain-Inhibitoren; Ribosomen-inaktivierende Proteine (RIPs), z. B. Ricin, Mais-RIP, Abrin, Luffin, Saporin oder Bryodin; Steroid-metabolisierende Enzyme, z. B. 3-Hydroxysteroid-Oxidase, Ecdysteroid-IDP-Glycosyl-Transferase, Cholesterinoxidase, Ecdyson-Inhibitoren oder HMG-CoA-Reduktase; Ionenkanalblocker, z. B. Inhibitoren von Natrium- oder Calziumkanälen; Juvenilhormon-Esterase; Rezeptoren für das diuretischen Hormon (Helicokininrezeptoren); Stilbensynthase, Bibenzylsynthase, Chitinasen und Glucanasen. Diese Toxine können in den Pflanzen auch als Prätoxine, Hybridproteine, verkürzte oder anderweitig modfizierte Proteine produziert werden. Hybridproteine zeichnen sich durch eine neue Kombination von verschiedenen Proteindomänen aus (siehe z. B. WO 2002/015701). Weitere Besipiele für derartige Toxine oder gentechnisch veränderte Pflanzen, die diese Toxine produzieren, sind in EP-A 374 753,
WO 93/07278, WO 95/34656, EP-A 427 529, EP-A 451 878, WO 03/18810 und
WO 03/52073 offenbart. Die Methoden zur Herstellung dieser gentechnisch veränderten Pflanzen sind dem Fachmann bekannt und z. B. in den oben erwähnten Publikationen dargelegt. Zahlreiche der zuvor genannten Toxine verleihen den Pflanzen, die diese produzieren, eine Toleranz gegen Schädlinge aus allen taxonomischen Arthropodenklassen, insbesondere gegen Käfer (Coeleropta), Zweiflügler (Diptera) und Schmetterlinge (Lepidoptera) und gegen Nematoden (Nematoda). Gentechnisch veränderte Pflanzen, die ein oder mehrere Gene, die für insektizide Toxine kodieren, produzieren sind z. B. in den oben erwähnten Publikationen beschrieben und zum Teil kommerziell erhältlich, wie z. B. YieldGard^{®} (Maissorten, die das Toxin Cry1Ab produzieren), YieldGard^{®} Plus (Maissorten, die die Toxine Cry1Ab und Cry3Bb1 produzieren), Starlink^{®} (Maissorten, die das Toxin Cry9c produzieren), Herculex^{®} RW (Maissorten, die die Toxine Cry34Ab1, Cry35Ab1 und das Enzym Phosphinothricin-N-Acetyltransferase [PAT] produzieren); NuCOTN^{®} 33B (Baumwollsorten, die das Toxin Cry1Ac produzieren), Bollgard^{®} I (Baumwollsorten, die das Toxin Cry1Ac produzieren), Bollgard^{®} II (Baumwollsorten, die die Toxine Cry1Ac und Cry2Ab2 produzieren); VIP-COT^{®} (Baumwollsorten, die ein VIP-Toxin produzieren); NewLeaf^{®} (Kartoffelsorten, die das Toxin Cry3A produzieren); Bt-Xtra^{®}, NatureGard^{®}, KnockOut^{®}, BiteGard^{®}, Protecta^{®}, Bt11 (z. B. Agrisure^{®} CB) und Bt176 von Syngenta Seeds SAS, Frankreich, (Maissorten, die das Toxin Cry1Ab und das PAT-Enyzm produzieren), MIR604 von Syngenta Seeds SAS, Frankreich (Maissorten, die ein modifizierte Version des Toxins Cry3A produzieren, siehe hierzu WO 03/018810), MON 863 von Monsanto Europe S.A., Belgien (Maissorten, die das Toxin Cry3Bb1 produzieren), IPC 531 von Monsanto Europe S.A., Belgien (Baumwollsorten, die eine modifizierte Version des Toxins Cry1Ac produzieren) und 1507 von Pioneer Overseas Corporation, Belgien (Maissorten, die das Toxin Cry1F und das PAT-Enyzm produzieren).

Weiterhin sind auch Pflanzen umfasst, die mit Hilfe gentechnische Maßnahmen ein oder mehrere Proteine produzieren, die eine erhöhte Resistenz oder Widerstandfähigkeit gegen bakterielle, virale oder pilzliche Pathogene bewirken, wie z. B. sogenannte Pathogenesis-related-Proteine (PR-Proteine, siehe EP-A 0 392 225), Resistenzproteine (z. B. Kartoffelsorten, die zwei Resistenzgene gegen *Phytophthora infestans* aus der mexikanischen Wildkartoffel *Solanum bulbocastanum* produzieren) oder T4-Lysozym (z. B. Kartoffelsorten, die durch die Produktion diese Proteins resistent gegen Bakterien wie *Erwinia amylvora* sind).

Weiterhin sind auch Pflanzen umfasst, deren Produktivität mit Hilfe gentechnischer Methoden verbessert wurde, indem z. B. die Ertragsfähigkeit (z. B. Biomasse, Kornertrag, Stärke-, Öl- oder Proteingehalt), die Toleranz gegenüber Trockenheit, Salz oder anderen begrenzenden Umweltfaktoren oder die Widerstandsfähigkeit gegenüber Schädlingen und pilzlichen, bakteriellen und viralen Pathogenen gesteigert wird. Weiterhin sind auch Pflanzen umfasst, deren Inhaltsstoffe insbesondere zur Verbesserung der menschlichen oder tierischen Ernährung mit Hilfe gentechnischer Methoden verändert wurden, indem z. B. Ölpflanzen gesundheitsfördernde langkettige Omega-3-Fettsäuren oder einfach ungesättigte Omega-9-Fettsäuren (z. B. Nexera^{®}-Raps, DOW Agro Sciences, Kanada) produzieren.

Die vorliegende Erfindung betrifft auch Saatgut (wie Saaten oder andere pflanzliche Vermehrungsmaterialien) enthaltend die erfindungsgemäße Zusammensetzung. Pflanzliche Vermehrungsmaterialien können vorbeugend zusammen mit oder bereits vor der Aussaat bzw. zusammen mit oder bereits vor dem Umpflanzen mit der erfindungsgemäßen Zusammensetzung behandelt werden. Für die Behandlung von Saatgut werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gele (GF) verwendet. Diese Zusammensetzungen können auf die Vermehrungsmaterialien, insbesondere Saatgut, unverdünnt oder, bevorzugt, verdünnt angewendet werden. Hierbei kann die entsprechende Zusammensetzung 2 bis 10fach verdünnt werden, so dass in den für die Beize zu verwendeten Zusammensetzungen 0,01 to 60 Gew.%, vorzugsweise 0,1 to 40 Gew. % Wirkstoff vorhanden sind. Die Anwendung kann vor oder während der Aussaat erfolgen. Die Behandlung von pflanzlichem Vermehrungsmaterial, insbesondere die Behandlung von Saatgut, ist dem Fachmann bekannt, und erfolgen durch Bestäuben, Beschichten, Pelletieren, Eintauchen oder Tränken des pflanzlichen Vermehrungsmaterial, wobei die Behandlung bevorzugt durch Pelletieren, Beschichten und Bestäuben oder durch Furchenbehandlung erfolgt, so dass z. B. eine vorzeitige Keimung des Saatguts verhindert wird. Für die Saatgutbehandlung werden bevorzugt Suspensionen verwendet. Üblicherweise enthalten solche Zusammensetzungen 1 bis 800 g/l Wirkstoff, 1 bis 200 g/l Tenside, 0 bis 200 g/l Frostschutzmittel, 0 bis 400 g/l Bindemittel, 0 bis 200 g/l Farbstoffe und Lösungsmittel, vorzugsweise Wasser.

Die vorliegende Erfindung betrifft weiterhin ein Alkoxylat, wobei das Alkoxylat ein Aminalkoxylat (A) 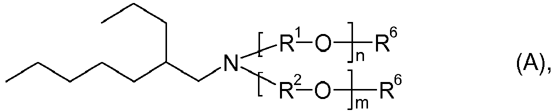 oder ein quartärnisiertes Derivat (AQ) 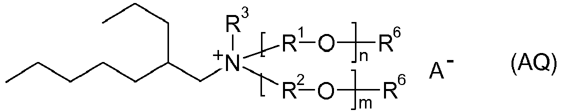 des Aminalkoxylats (A) ist, wobei
R¹, R², und R⁵ unabhängig voneinander Ethylen, Propylen, Butylen oder eine Mischung davon sind,
R³ ein H, -OH, -OR⁴, -[R⁵-O]ₚ-R⁶, C₁-C₆ Alkyl oder ein Sauerstoffanion,
R⁴ ein C₁-C₆ Alkyl, C₂-C₆ Alkenyl oder C₂-C₆ Alkinyl,
R⁶ ein H, C₁-C₆ Alkyl, C₂-C₆ Alkenyl, C₂-C₆ Alkinyl, -SO₃R^{a}, -P(O)OR^{b}OR^{c}, -CH₂CO₂R^{d}, oder -C(O)R^{e},
R^{a} und R^{d} unabhängig voneinander ein H, anorganische oder organische Kationen,
R^{b} und R^{c} unabhängig voneinander ein H, anorganische oder organische Kationen, C₁-C₆ Alkyl, C₂-C₆ Alkenyl oder C₂-C₆ Alkinyl,
R^{e} C₁-C₂₂-Alkyl, C₂-C₂₂-Alkenyl, C₂-C₂₂-Alkinyl, C₆-C₂₂-Aryl, C₇-C₂₂-Alkylaryl,
n, m und p unabhängig voneinander ein Wert von 1 bis 30,
A- ein landwirtschaftlich akzeptables Anion, oder, falls R³ ein Sauerstoffanion ist, A-nicht vorhanden ist. Bevorzugte Parameter sind wie oben beschrieben.

In einer Ausführungsform ist das Alkoxylat ein quartärnisiertes Derivat (AQ) des Aminalkoxylat (A). In einer bevorzugten Ausführungsform ist das Alkoxylat ein Aminalkoxylat (A).

In einer weiteren bevorzugten Ausführungsform ist das Alkoxylat ein quartärnisiertes Derivat (AQ) des Aminalkoxylat (A). Dabei ist A⁻ bevorzugt ein Halogenid (wie Chlorid oder Bromid), Phosphat, Sulfat oder ein anionisches Pestizid. Besonders bevorzugt ist A⁻ ein anionisches Pestizid, wie Glyphosatanion oder Glufosinatanion.

Die vorliegende Erfindung betrifft weiterhin Verfahren zur Herstellung des Aminalkoxylats (A) oder eines quartärnisierten Derivats (AQ) des Aminalkoxylats (A) umfassend die Alkoxylierung von 2-Propylheptylamin mit Ethylenoxid, Propylenoxid, Butylenoxid oder eine Mischung davon. Die Herstellung von 2-Propylheptylamin ist allgemein bekannt, beispielsweise durch Umsetzung von Ammoniak mit 2-Propylheptanol wie in US 5,808,158 beschrieben.

Die Alkoxylierung kann durch starke Basen, wie Alkalihydroxide und Erdalkalihydroxide, Brönstedsäuren oder Lewissäuren, wie AlCl₃, BF₃. katalysiert werden. Für eng verteilte Alkoholalkoxylate können Katalysatoren wie Hydrotalcit oder DMC verwendet werden. Die Alkoxylierung erfolgt vorzugsweise bei Temperaturen im Bereich von etwa 80 bis 250 °C, bevorzugt etwa 100 bis 220 °C. Der Druck liegt vorzugsweise zwischen Umgebungsdruck und 600 bar. Gewünschtenfalls kann das Alkylenoxid eine Inertgasbeimischung, z. B. von etwa 5 bis 60 %, enthalten.

Das quartärnisierte Derivats (AQ) des Aminalkoxylats (A) kann in einem weiteren Reaktionsschritt aus dem Aminalkoxylat (A) durch Quartärnisierung hergestellt werden. Zur Einführung des Restes R³ in das Aminalkoxylat (A) kann es beispielsweise mit einem Alkylierungsreagenz wie Methylchlorid, Dimethylsulfat, oder Butylchlorid umgesetzt werden. Zur Einführung des eines Sauerstoffanions in das Aminalkoxylat (A) kann es oxidiert werden, beispielsweise durch Umsetzung der Aminogruppe mit Wasserstoffperoxid, Persäuren (wie meta-Chlorperbenzoesäure oder Peressigsäure) oder Peroxomonoschwefelsäure.

Die Herstellung der quartärnisiertes Derivate (AQ) mit R³ = H kann durch einfache Protonierung von Ausgangsverbindungen der Struktur (A) erfolgen. Die Herstellung der quartärnisiertes Derivate (AQ) mit R³ = OH kann durch einfache Protonierung von Ausgangsverbindungen (AQ) mit R³ = Sauerstoffanion erfolgen. Zur Protonierung sind als Säuren organische Säuren (z.B. C₁ bis C₂₀ Carbonsäuren, insbesondere Benzoesäure) oder anorganische Säure (z.B. Salzsäure, Phosphorsäure, oder Schwefelsäure) geeignet. Ebenso geeignet sind auch H-azide Pestizide wie z.B. Glyphosate-säure oder Glyphosate-monosalze. Die Protonierung kann in einem separaten Syntheseschritt durchgeführt werden, so dass das quartärniesierte Derivat (AQ) isoliert werden kann. Es ist auch möglich die Protonierung durch Mischen der Ausgangsverbindungen mit einer oder mehreren Säuren in der Zusammensetzung oder in die Spritzbrühe durchzuführen.

Die vorliegende Erfindung betrifft auch die Verwendung des Aminalkoxylats (A) oder eines quartärnisierten Derivats (AQ) des Aminalkoxylats (A) wie oben beschrieben als Hilfsmittel in Pestizid-haltigen Spritzbrühen. Bevorzugt ist das Hilfsmittel ein wirkungssteigerndes Hilfsmittel. Solche Wirkungssteigernden Hilfsmittel werden auch Adjuvantien genannt. Sie steigern oder beschleunigen die Wirkung von Pestiziden im Vergleich zur Wirkung des Pestizides in Abwesenheit des Adjuvants.

Die Vorteile der Erfindung sind eine hohe Stabilität der Formulierung und der Spritzbrühe, ein geringer Winddrift bei Sprühapplikation, eine hohe Haftung der Formulierung auf der Oberfläche der behandelten Pflanzen, eine Erhöhung der Löslichkeit der Pestizide in der Formulierung, eine erhöhte Permeation der Pestizide in die Pflanze und damit verbunden eine schnellere und erhöhte Wirksamkeit. Ein wichtiger Vorteil ist die geringe Toxizität der neuen Alkoxlyate, insbesondere die geringe Aquatoxizität. Ein Vorteil ist auch die geringe Schadwirkung gegen Kulturpflanzen, d.h. eine geringe phytotoxische Effekte. Ein weiterer Vorteil liegt in der einfachen Handhabung dieser Alkoxide, da z.B. in der Einarbeitung in Formulierungen keine Gelbildung auftritt.

Nachfolgende Beispiele erläutern die Erfindung ohne sie einzuschränken.

### Beispiele

### Beispiel 1 - Synthese 2-Propylheptylamin (2-PHamin)

2-Propylheptanol (kommerziell erhältlich von BASF SE oder Evonik) und ein Alkohol-Aminierungskatalysator (beschrieben in EP 696 572 A1; 8 Gew.% bezüglich 2-Propylheptanol) wurdem im Autoklaven vorgelegt und es wurde mit Stickstoff und Wasserstoff gespült. Dann wurde Ammoniak beaufschlagt im Molverhältnis Ammoniak zu Alkohol von 17 zu 1 und unter Rühren erwärmt. Nach einer Reaktionszeit von zehn Stunden bei 210 °C und einem Druck H₂ von 240 bar wurde das 2-Propylheptylamin filtriert und am Rotationsverdampfer von Wasser befreit.

### Beispiel 2 - Alkoxilierung von 2-Propylheptylamin

### A) Herstellung von 2-PHamin-7 EO

Zunächst wurden 628 g (4 mol) 2-Propylheptylamin aus Beispiel 1 mit 19,6 g Wasser versetzt. Dann wurde nach Spülen mit Stickstoff bei 100 °C 352 g (8 mol) Ethylenoxid (EO) zudosiert. Anschließend wurde bei 80°C unter Vakuum entwässert. Man erhielt eine Ausbeute von 988g (=99% d.T.) mit einer OH-Zahl von 468 mg KOH/g (458 mgKOH/g Theorie) und einer Amin-Zahl von 229 mg KOH/g (229 mg KOH/g Theorie). Im zweiten Schritt wurden zu 269,5 g (1,1 mol) von diesem Vorprodukt 2,0 g 50%ige KOH zugegeben und bei 90°C unter Vakuum entwässert. Nach Spülen mit Stickstoff wurden bei 120°C 242 g (=5,5 mol) Ethylenoxid zudosiert. Das Produkt wurde mit wenigen Tropfen Essigsäure auf pH (5 % in Wasser) 9,9 eingestellt. Man erhielt eine Ausbeute von 512 g (100% d.T.) einer gelblichen niedrigviskose Flüssigkeit (OH-Zahl: 279,1 mg KOH/g (241 mg KOH/g Theorie); Amin Zahl: 120 mg KOH/g (121 mg KOH/g Theorie), Wassergehalt <0,5 wt%).

### B) Herstellung von 2-PHamin-10 EO

Zunächst wurden 1280 g (8,15 mol) 2-Propylheptylamin aus Beispiel 1 mit 40 g Wasser versetzt. Dann wurde nach Spülen mit Stickstoff bei 100 °C 717 g (16,3 mol) Ethylenoxid zudosiert (2 bar, 16 h). Anschließend wurde bei 90°C unter Vakuum restliche Spuren von Ethylenoxid entfernt. Man erhielt eine quantitative Ausbeute mit einer Amin-Zahl von 229 mg KOH/g).
Im zweiten Schritt wurden zu 821,5 g (3,35 mol) von diesem Vorprodukt 8,0 g 50%ige KOH zugegeben und bei 90°C unter Vakuum entwässert. Nach Spülen mit Stickstoff wurden bei 120°C 1179 g (=26,8 mol) Ethylenoxid zudosiert (1,5 bar, 12 h). Man erhielt eine quantitative Ausbeute einer gelblichen niedrigviskose Flüssigkeit.

### C) bis F) Herstellung von 2-PHamin-5 EO, 2-PHamin-8,5 EO, 2-PHamin-15 EO, 2-PHamin-20 EO

Die Ethoxylierung von 2-Propylheptylamin aus Beispiel 1 wurde entsprechend Beispiel 2A und 2B durchgeführt. So konnte in quantitativer Ausbeute 2-PHamin-5 EO, 2-PHamin-8,5 EO, 2-PHamin-15 EO und 2-PHamin-20 EO synthetisiert werden. Dabei geben die Kürzel "-5 EO", "-8,5 EO usw. den molaren Überschuß von Ethylenoxid zu 2-Propylheptylamin wieder.

### G) Herstellung von 2-PHamin-Ethoxylat-Propoxylat

Allgemeine Herstellvorschrift: 2-Propylheptylamin (1 mol eq.; aus Beispiel 1) wurden im Reaktor mit Wasser (2 Gew.% auf dem Gesamtansatz) versetzt. Dann wurde nach Spülen mit Stickstoff und Einstellung des Drucks auf 1.5 bar bei 100°C-130°C Ethylenoxid (2 mol eq.) zudosiert. Anschließend wurde bei 80°C unter Vakuum entwässert. Die Ausbeute wird bestimmt sowie OH-Zahl und Amin-Zahl, um die Qualität des Vorproduktes zu bestimmen.
Im zweiten Schritt wurden zu diesem Vorprodukt (1 mol eq.) 50%ige KOH (0.2 Gew.% auf dem Gesamtansatz) zugegeben und bei 90°C unter Vakuum entwässert. Nach Spülen mit Stickstoff wird der Druck auf 1.5 bar eingestellt und bei 120°C-140°C Alkylenoxid (x mol eq. Ethylenoxid oder Propylenoxid oder eine Mischung aus Propylenoxid oder Ethylenoxid) zudosiert. Die Reaktionsmischung wurde bei dieser Temperatur nachgerührt bis zur Druckkonstanz.
Entweder wurde das Produkt ausgebaut und mit Eisessig auf pH 9,9 (5% in Wasser) eingestellt oder wird bei derselben Temperatur (120-140°C) Propylenoxid (y mol eq.) zudosiert. Die Reaktionsmischung wird bei dieser Temperatur nachgerührt bis zur Druckkonstanz. Das Produkt wird isoliert, mit Eisessig auf pH 9,9 (5% in Wasser) eingestellt.

Die Alkoxylierung von 2-Propylheptylamin zum 2-PHamin-5 EO-15 PO wurde entsprechend der allgemeinen Herstellvorschrift durchgeführt. Dabei wurde im ersten Schritt mit 2 molarem Überschuß Ethylenoxid umgesetzt und im zweiten Schritt das restliche Ethylenoxid und Propylenoxid dazugegeben. Man erhielt in quantitativer Ausbeute 2-PHamin-5 EO-15 PO.

### H bis Q) Herstellung von 2-PHamin-Propoxylat-Ethoxylat

Allgemeine Herstellvorschrift: 2-Propylheptylamin (1 mol eq.) wurden im Reaktor mit Wasser (2 Gew. % auf dem Gesamtansatz) versetzt. Dann wurde nach Spülen mit Stickstoff und Einstellung des Drucks auf 1.5 bar bei 100°C-130°C Propylenoxid (2 mol eq.) zudosiert. Anschließend wurde bei 80°C unter Vakuum entwässert. Die Ausbeute wurde bestimmt sowie OH-Zahl und Amin-Zahl, um die Qualität des Vorproduktes zu bestimmen.

Im zweiten Schritt wurden zu diesem Vorprodukt (1 mol eq.) 50%ige KOH (0.2 Gew. % auf dem Gesamtansatz) zugegeben und bei 90°C unter Vakuum entwässert. Nach Spülen mit Stickstoff wird der Druck auf 1.5 bar eingestellt und wird bei 120°C-140°C Alkylenoxid (x mol eq. Ethylenoxid oder Propylenoxid oder eine Mischung aus Propylenoxid oder Ethylenoxid) zudosiert. Die Reaktionsmischung wird bei dieser Temperatur nachgerührt bis zur Druckkonstanz.

Entweder wurde das Produkt ausgebaut und mit Eisessig auf pH 9,9 (5% in Wasser) eingestellt oder es wurde bei derselben Temperatur (120-140°C) Propylenoxid (y mol eq.) zudosiert. Die Reaktionsmischung wurde bei dieser Temperatur nachgerührt bis zur Druckkonstanz. Das Produkt wurde isoliert, und mit Eisessig auf pH 9,9 (5% in Wasser) eingestellt.

Nach dieser Vorschrift wurden die folgenden Alkoxylate mit jeweils quantitativer Ausbeute hergestellt:
I) 2-PHamin-2 PO-5 EO
J) 2-PHamin-2 PO-10 EO
K) 2-PHamin-2 PO-15 EO
L) 2-PHamin-4 PO-5 EO
M) 2-PHamin-4 PO-10 EO
N) 2-PHamin-4 PO-15 EO
O) 2-PHamin-6 PO-5 EO
P) 2-PHamin-6 PO-10 EO
Q) 2-PHamin-6 PO-15 EO

### Beispiel 3 - Glyphosat SL Formulierung gegen Brachiarea multica

In einem Feldversuch wurden sieben Versuchsflächen mit je sechs Quadrameter und jeweils in zweifacher Ausfertigung (d.h. zwei Parzellen mit je 3 m²) das Unkraut *Brachiarea multica* behandelt. Dazu wurde jeweils 1,0 I Sprühlösung (Siehe Tabelle 1) pro Parzelle mit einem Handsprühapparat versprüht. 1,0 I der Sprühlösung enthielten 7,5 ml einer wässerigen Formulierung von gelöstem Glyphosat-Isopropylammonium Salz. Zur Herstellung dieser Formulierung wurden zuvor 100 ml Aminalkoxylat aus Beispiel 2 auf 1,0 I aufgefüllt mit 46%iger Glyphosate-Isopropylammonium Salz Lösung. Zwei, fünf und sieben Tagen nach der Applikation wurde visuell abgeschätzt, wieviel Prozent des Unkrauts vernichtet worden waren. In den Tagen zwischen Applikation und Bewertung kam es wiederholt zu Regenfällen.

Zum Vergleich wurde eine Glyphosatformulierung wie oben, aber ohne Aminalkoxylat hergestellt, sowie eine kommerzielle Glyphosatformulierung (Roundup®, Monsanto) verwendet, welche 41,5 Gew.% Glyphosat-Isopropylammoniumsalz und 15,5 Gew.% ethoxyliertes Talgfettamin mit etwa 15 mol Ethylenoxid pro Amingruppe enthielt ("Talgfettamin--15 EO").

**Tabelle 1: Anteil [%] an vernichtetem Unkraut**

| Aminalkoxylat | 2 Tage | 5 Tage | 7 Tage |
|---|---|---|---|
| - ^{a)} | 15 | 35 | 55 |
| Talgfettamin--15 EO ^{a)} | 40 | 70 | 75 |
| 2-Ethylhexylamin-7 EO ^{a)} | 25 | 60 | 77,5 |
| Isotridecylamin-9 EO ^{a)} | 20 | 40 | 62 |
| 2-PHamin-7 EO (aus Beispiel 2) | 40 | 87,5 | 90 |

| | | | |
|---|---|---|---|
| a) Vergleichsversuch, nicht erfindungsgemäß. | | | |

Die Daten in Tabelle 1 zeigten, dass das erfindungsgemäße 2-Propylheptylaminethoxylat ("2-PHamin-7 EO") die Wirkung von Glyphosat erhöht und beschleunigt. Auch die Regenfestigkeit der Formulierung war gegeben. Die Formulierung war bei Temperaturen von -5 bis +55 °C für mindestens zwei Wochen lagerstabil.

Beim Zugeben von 2-PHamin-7 EO bei 20 °C in die Glyphosat-Lösung kam es vorteilhafterweise zu keiner Gelbildung, wie es sonst beim Zugeben von Talgfettamin--15 EO auftrat.

### Beispiel 4 -Wassertoxizität

- 2-PHamin-7 EO (aus Beispiel 2): EC50 60 mg/l (48 h) an *Daphnia magna*, bestimmt nach OECD-Richtlinie 202 Teil 1.
- 2-PHamin-10 EO (aus Beispiel 2): Fischtoxizität LC50 >100 mg/l (96 h), *Brachydanio rerio,* bestimmt nach OECD 203; ISO 7346; 84/449/EEC, C.1.; EC50 (72 h) > 100 mg/l, Algen, bestimmt nach OECD Guideline 201.
- Lutensol® FA15 T (Talgfettaminethoxylat mit 15 EO): EC50 2,6 mg/l (48 h) an *Daphnia magna*, bestimmt nach OECD-Richtlinie 202 Teil 1; Fischtoxizität LC50 1-10 mg/l (96 h), *Leuciscus idus* (Sicherheitsdatenblatt vom 8.3.2006 von BASF SE).
- Lutensol® FA 12 (Oleylaminethoxylat mit 12 EO): EC50 0,1-1 mg/l (48 h) an *Daphnia magna*, Fischtoxizität LC50 1-10 mg/l (96 h), *Leuciscus idus* (Sicherheitsdatenblatt vom 18.8.2006 von BASF SE).

### Beispiel 5 - Glyphosat SL Formulierung auf Raps

Für die Gewächshaustests wurde Winterraps (Sorte Remy) in lehmigem Sandboden mit einer Aussaattiefe von 1-2 cm ausgesät bzw. getopft. Sobald die Pflanzen eine Wuchshöhe von 10 bis 25 cm (d.h. ca. 10 bis 21 Tage nach der Aussaat) erreicht hatten, wurden die Spritzbrühen auf die Pflanzen in der Spritzkabine appliziert.

Eine konzentrierte Formulierung enthaltend Glyphosat Isoproylammonium gelöst in Wasser und Aminalkoxylat aus Beispiel 2 wurde mit VE-Wasser verdünnt und wurden mit einer Wasseraufwandmenge von 375 l/ha ausgebracht (140 g Glyphosat/ha und 300 g Aminalkoxylat/ha). Die Temperaturen in der 3 bis 4 Wochen dauernden Versuchsperiode betrugen zw. 18 - 35 ° C. Während dieser Zeit wurden die Versuchspflanzen optimal bewässert, wobei die Nährstoffversorgung über das Gießwasser erfolgte.

Die Bewertung der herbiziden Aktivität erfolgte mittels Bonitur der behandelten Pflanzen im Vergleich zu den unbehandelten Kontrollpflanzen (Tabelle 2). Die Bewertungsskala reicht von 0 % bis 100 % Wirkung. 100 % Wirkung bedeutet vollständiges Absterben zumindest der oberirdischen Pflanzenteile. Demgegenüber bedeutet 0 % Wirkung, das keine Unterschiede zwischen behandelten und unbehandelten Pflanzen auftraten. Die Ergebnisse in Tabelle 2 belegen die erhöhte Wirksamkeit des Wirkstoffs durch Zusatz des Aminalkoxylats.

**Tabelle 2:**

| Aminalkoxylat | Wirksamkeit [%] nach 14 Tagen | Wirksamkeit [%] nach 21 Tagen |
|---|---|---|
| - ^{a)} | 51 | 69 |
| 2-PHamin-5 EO | 59 | 74 |
| 2-PHamin-7 EO | 75 | 86 |
| 2-PHamin-10 EO | 74 | 86 |
| 2-PHamin-15 EO | 63 | 74 |
| 2-PHamin-20 EO | 63 | 74 |
| 2-PHamin-5 EO-15 PO | 63 | 74 |
| N-Methyl-2-PHamin-7 Eo ^{a), b)} | 56 | 65 |

| | | |
|---|---|---|
| a) Vergleichsversuch, nicht erfindungsgemäß. b) Hergestellt analog WO 2009/004044, durch Addition von Methylamin an das entsprechende Enon, welches dann analog Beispiel 2A ethoxyliert wurde. Das so erhaltene neutrale N-Methylamin (nicht quarternisiert) hatte eine Aminzahl von 117,8 mg/KOH/g). | | |

### Beispiel 6 - Glyphosat SL Formulierung auf Weizen, Soja oder Mais

Die Versuche wurden wie in Beispiel 5 durchgeführt an Winterweizen (Sorte Cubus), Soja (Sorte Oxford) und Mais (Sorte Amadeo). Die Aufwandmenge betrug 280 g Glyphosat/ha und 300 g Aminalkoxylat/ha. Die Ergebnisse in Tabelle 3 belegen die erhöhte Wirksamkeit des Wirkstoffs durch Zusatz des Aminalkoxylats.

**Tabelle 3: Wirksamkeit [%] nach 21 Tagen**

| Aminalkoxylat | Winterweizen | Soja | Mais |
|---|---|---|---|
| - ^{a)} | 73 | 38 | 54 |
| 2-PHamin-8,5 EO | 96 | 93 | 89 |
| 2-Ethylhexylamin-7 EO ^{a)} | 84 | 51 | 71 |

| | | | |
|---|---|---|---|
| a) Vergleichsversuch, nicht erfindungsgemäß. | | | |

### Beispiel 7 - Glyphosat SL Formulierung auf Raps

Die Versuche wurden wie in Beispiel 5 durchgeführt an Winterraps (Sorte Remy). Die Aufwandmenge betrug 140 g Glyphosat/ha und 300 g Aminalkoxylat/ha. Die Ergebnisse in Tabelle 4 belegen die erhöhte Wirksamkeit des Wirkstoffs durch Zusatz des Aminalkoxylats.

**Tabelle 4:**

| Aminalkoxylat | Wirksamkeit [%] nach 14 Tagen | Wirksamkeit [%] nach 21 Tagen |
|---|---|---|
| - ^{a)} | 51 | 69 |
| 2-PHamin-2 PO-5 EO | 94 | 97 |
| 2-PHamin-2 PO-10 EO | 92 | 91 |
| 2-PHamin-2 PO-15 EO | 97 | 98 |
| 2-PHamin-4 PO-5 EO | 86 | 86 |
| 2-PHamin-4 PO-10 EO | 92 | 91 |
| 2-PHamin-4 PO-15 EO | 94 | 93 |
| 2-PHamin-6 PO-5 EO | 83 | 80 |
| 2-PHamin-6 PO-10 EO | 86 | 84 |
| 2-PHamin-6 PO-15 EO | 91 | 86 |

| | | |
|---|---|---|
| a) Vergleichsversuch, nicht erfindungsgemäß. | | |

### Beispiel 8 - Imazamox SL Formulierung auf Ambrosia und Chenopodium

Die Versuche wurden wie in Beispiel 5 durchgeführt an *Ambrosia artemisiifolia* (AMBEL) bzw. *Chenopodium album* (CHEAL) und nach 14 Tagen bonitiert. Die Spritzbrühe wurde ausgehend von einer wässsrigen SL Formulierung hergestellt, die 120 g/l Imazamox Ammoniumsalz und 1,2-Propylenglykol enthielt. Die Aufwandmenge betrug 10 g Imazamox/ha und 400 g Aminalkoxylat/ha. Die Ergebnisse sind in Tabelle 5 zusammengefasst. Als Vergleich wurde ein Talgfettaminethoxylat mit 12 Ethylenoxideinheiten eingesetzt (Flüssigkeit, ca. 100 % Gehalt, Molmasse ca 730 g/mol (berechnet von OH-Zahl), kommerziell erhältlich als Lutensol® FA12K von BASF SE).

**Tabelle 5:**

| Aminalkoxylat | Wirksamkeit [%] AMBEL | Wirksamkeit [%] CHEAL |
|---|---|---|
| Lutensol® FA12K ^{a)} | 80 | 90 |
| 2-PHamin-5 EO | 80 | 93 |
| 2-PHamin-7 EO | 80 | 93 |
| 2-PHamin-10 EO | 83 | 90 |
| 2-PHamin-15 EO | 80 | 93 |
| 2-PHamin-20 EO | 85 | 93 |

| | | |
|---|---|---|
| a) Vergleichsversuch, nicht erfindungsgemäß. | | |

### Beispiel 9 - Imazaquin, Chlormequatchlorid und Cholinchlorid SL Formulierung zur Wachstumsregulierung auf Weizen

Die Versuche wurden wie in Beispiel 5 durchgeführt an Weizen. Die Höhe der Halme wurde 7, 14 und 21 Tage nach Applikation gemessen. Die Spritzbrühe wurde ausgehend von einer wässsrigen SL Formulierung hergestellt, die 0,8 g/l Imazaquin, 368 g/l Chlormequatchlorid, 28 g/l Cholinchlorid, und 80 g/l Aminalkoxylat aus Beispiel 2 C (2-PHamin-10 EO) enthielt. Die Aufwandmenge betrug 1500 g/ha, 1000 g/ha bzw. 500 g/ha Chlormequatchlorid. Die Ergebnisse sind in Tabelle 6 zusammengefasst.

**Tabelle 6: Höhe der Weizenhalme [cm] 7, 14 und 21 Tage nach Applikation**

| Aufwandmenge Chlormequatchlorid | 7 Tage | 14 Tage | 21 Tage |
|---|---|---|---|
| - ^{a)} | 30,1 | 32,5 | 39,3 |
| 1500 g/ha | 25,1 | 25,1 | 26,4 |
| 1000 g/ha | 25,8 | 27,1 | 27,0 |
| 500 g/ha | 22,3 | 27,4 | 26,9 |

| | | | |
|---|---|---|---|
| a) unbehandelter Weizen, Vergleichsversuch, nicht erfindungsgemäß. | | | |

### Beispiel 10 - Regenfestigkeit

Jeweils vier Pflanzen Mais bzw. Winterweizen wurden mit 280 /ha Glyphosat Isopropylammoniumsalz und 300 g/ha Aminalkoxylat behandelt. Nach 1,5 oder 3 h nach dieser Behandlung wurden die Pflanzen 20 min beregnet mit 100 L Wasser bei einem Druck von 3,33 bar und einer Geschwindigkeit von 2,8 m/s. Die Pflanzen wurden anschließend ins Gewächshaus gestellt und nach 14 bzw. 21 bonitiert wie in Beispiel 5. Zum Vergleich wurde Genamine® T 150 eingesetzt (C_{16/18}-aminethoxylat mit 15 EO Einheiten, kommerziell erhältlich von Clariant).
Tabelle 7 zeigt, dass durch die Beregnung das Glyphosat weniger stark von der Pflanze abgelöst wird, so dass die Wirksamkeit auch nach Beregnung hoch bleibt.

**Tabelle 7: Wirksamkeit [%] nach 21 Tagen**

| Aminalkoxylat | | Ohne Beregnung | Beregnung 3 h nach Applikation |
|---|---|---|---|
| Keine Applikation ^{a)} | Mais | 0 | - |
| Genamine® T 150 ^{a)} | Mais | 98 | 53 |
| 2-PHamin-2 10 EO | Mais | 98 | 71 |
| Keine Applikation ^{a)} | Weizen | 0 | - |
| Genamine® T 150 ^{a)} | Weizen | 100 | 94 |
| 2-PHamin-2 10 EO | Weizen | 100 | 97 |

| | | | |
|---|---|---|---|
| a) Vergleichsversuch, nicht erfindungsgemäß. | | | |

### Beispiel 11 - Wasserlösliches Granulat SG

Glyphosatemonoammoniumsalz (80,75 g) wurde mit Natriumsulfit (0,5g) in einer Kaffeemühle gut durchmischt. Anschließend wurde das Gemisch mit einer Lösung aus 18,7 g 2-PHamin-15 EO (aus Beispiel 2) und 4 g Wasser angeteigt und extrudiert (Benchtop, 0,8 mm Sieb). Das Extrudat wurde anschließend getrocknet und zerkleinert, um eine möglichst homogene Granulatgröße zu erhalten(durchschnittliche Länge ca. 1 mm). Das so erhaltene wasserlösliche Glyphosate-Granulat (SG) war physikalisch und chemisch stabil. Durch Auflösen in Wasser liessen sich daraus auf einfache Weise Spritzbrühen (z.B. 2%ig) mit hoher Qualität und Stabilität herstellen.

## Patentansprüche

1. Zusammensetzung umfassend ein Pestizid und ein Alkoxylat, **dadurch gekennzeichnet, dass** das Alkoxlyat ein Aminalkoxylat (A) 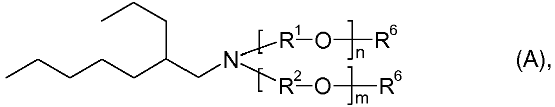 oder ein quartärnisiertes Derivat (AQ) 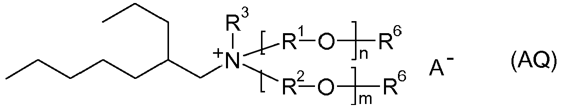 des Aminalkoxylats (A) ist, wobei
R¹, R², und R⁵ unabhängig voneinander Ethylen, Propylen, Butylen oder eine Mischung davon sind,
R³ ein H, -OH, -OR⁴, -[R⁵-O]ₚ-R⁶, C₁-C₆ Alkyl oder ein Sauerstoffanion,
R⁴ ein C₁-C₆ Alkyl, C₂-C₆ Alkenyl, oder C₂-C₆ Alkinyl,
R⁶ ein H, C₁-C₆ Alkyl, C₂-C₆ Alkenyl, C₂-C₆ Alkinyl, -SO₃R^{a}, -P(O)OR^{b}OR^{c}, -CH₂CO₂R^{d}, oder -C(O)R^{e},
R^{a} und R^{d} unabhängig voneinander ein H, anorganische oder organische Kationen,
R^{b} und R^{c} unabhängig voneinander ein H, anorganische oder organische Kationen, C₁-C₆ Alkyl, C₂-C₆ Alkenyl oder C₂-C₆ Alkinyl,
R^{e} C₁-C₂₂-Alkyl, C₂-C₂₂-Alkenyl, C₂-C₂₂-Alkinyl, C₆-C₂₂-Aryl, C₇-C₂₂-Alkylaryl,
n, m und p unabhängig voneinander ein Wert von 1 bis 30,
A- ein landwirtschaftlich akzeptables Anion, oder, falls R³ ein Sauerstoffanion ist, A⁻ nicht vorhanden ist.

2. Zusammensetzung nach Anspruch 1, wobei R¹, R² und R⁵ unabhängig voneinander Ethylen, Ethylen und Propylen, Ethylen und Butylen, oder Ethylen, Propylen und Butylen sind.

3. Zusammensetzung nach Anspruch 1 oder 2, wobei A⁻ ein Halogenid, Phosphat, Sulfat oder anionisches Pestizid ist.

4. Zusammensetzung nach einem der Ansprüche 1 bis 3, wobei R³ ein H ist.

5. Zusammensetzung nach einem der Ansprüche 1 bis 4, wobei beim Aminalkoxlat (A) die Summe aus n und m 2 bis 40, und bei dessen quartärnisiertes Derivat (AQ) die Summe aus n, m und p 3 bis 80.

6. Zusammensetzung nach einem der Ansprüche 1 bis 5, wobei das Alkoxylat das Aminalkoxylat (A) ist.

7. Zusammensetzung nach einem der Ansprüche 1 bis 6, wobei das Pestizid ein Pestizid mit mindestens einer H-aciden Gruppe umfasst.

8. Zusammensetzung nach einem der Ansprüche 1 bis 7, wobei das Pestizid Glyphosat oder Glufosinat, und zusätzlich ein weiteres Pestizid umfasst.

9. Aminalkoxylat (A) oder ein quartärnisiertes Derivat (AQ) des Aminalkoxylats (A) gemäß einem der Ansprüche 1 bis 5.

10. Aminalkoxylat (A) oder ein quartärnisiertes Derivat (AQ) des Aminalkoxylats (A) nach Anspruch 9, wobei R¹, R² und R⁵ unabhängig voneinander Ethylen, Ethylen und Propylen, Ethylen und Butylen, oder Ethylen, Propylen und Butylen sind; wobei R³ und R⁶ jeweils ein H sind; und wobei beim Aminalkoxlat (A) die Summe aus n und m 2 bis 40, und bei dessen quartärnisiertes Derivat (AQ) die Summe aus n, m und p 3 bis 80 ist.

11. Verfahren zur Herstellung des Aminalkoxylats (A) oder eines quartärnisierten Derivats (AQ) des Aminalkoxylats (A) gemäß einem der Ansprüche 1 bis 5 umfassend die Alkoxylierung von 2-Propylheptylamin mit Ethylenoxid, Propylenoxid, Butylenoxid oder eine Mischung davon.

12. Verfahren nach Anspruch 11, wobei R¹, R² und R⁵ unabhängig voneinander Ethylen, Ethylen und Propylen, Ethylen und Butylen, oder Ethylen, Propylen und Butylen sind; wobei R³ und R⁶ jeweils ein H sind; und wobei beim Aminalkoxlat (A) die Summe aus n und m 2 bis 40, und bei dessen quartärnisiertes Derivat (AQ) die Summe aus n, m und p 3 bis 80 ist.

13. Verfahren zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung gemäß einem der Ansprüche 1 bis 8 auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt.

14. Saatgut enthaltend die Zusammensetzung gemäß einem der Ansprüche 1 bis 8.

15. Verwendung des Aminalkoxylats (A) oder eines quartärnisierten Derivats (AQ) des Aminalkoxylats (A) gemäß einem der Ansprüche 1 bis 5 als Hilfsmittel in Pestizid-haltigen Spritzbrühen.

16. Verwendung nach Anspruch 13, wobei das Hilfmittel ein wirkungssteigerndes Hilfsmittel ist.

## Claims

1. A composition comprising a pesticide and an alkoxylate, wherein the alkoxylate is an amine alkoxylate (A) 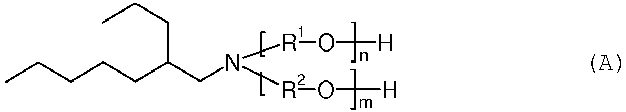 or a quaternized derivative (AQ) 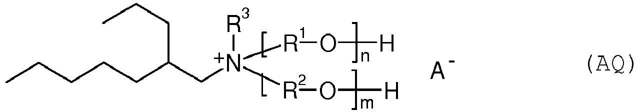 of the amine alkoxylate (A), where
R¹, R², and R⁵ independently of one another are ethylene, propylene, butylene or a mixture of these,
R³ is an H, -OH, -OR⁴, - [R⁵-O]ₚ-R⁶, C₁-C₆-alkyl or an oxygen anion,
R⁴ is a C₁-C₆-alkyl, C₂-C₆-alkenyl or C₂-C₆-alkynyl,
R⁶ is an H, C₁-C₆-alkyl, C₂-C₆-alkenyl, C₂-C₆-alkynyl, -SO₃R^{a}, -P(O)OR^{b}OR^{c}, -CH₂CO₂R^{d}, or -C(O)R^{e},
R^{a} and R^{d} independently of one another are an H, inorganic or organic cations,
R^{b} and R^{c} independently of one another are an H, inorganic or organic cations, C₁-C₆-alkyl, C₂-C₆-alkenyl or C₂-C₆-alkynyl,
R^{e} is C₁-C₂₂-alkyl, C₂-C₂₂-alkenyl, C₂-C₂₂-alkynyl, C₆-C₂₂-aryl or C₇-C₂₂-alkylaryl,
n, m and p independently of one another have a value of from 1 to 30,
A⁻ is an agriculturally acceptable anion, or, if R³ is an oxygen anion, A⁻ is absent.

2. The composition according to claim 1, wherein R¹, R² and R⁵ independently of one another are ethylene, ethylene and propylene, ethylene and butylene, or ethylene, propylene and butylene.

3. The composition according to claim 1 or 2, wherein A⁻ is a halide, phosphate, sulfate or anionic pesticide.

4. The composition according to any of claims 1 to 3, wherein R³ is an H.

5. The composition according to any of claims 1 to 4, wherein, in the amine alkoxylate (A), the total of n and m is from 2 to 40, and in its quaternized derivative (AQ) the total of n, m and p is from 3 to 80.

6. The composition according to any of claims 1 to 5, wherein the alkoxylate is the amine alkoxylate (A).

7. The composition according to any of claims 1 to 6, wherein the pesticide comprises a pesticide with at least one H-acidic group.

8. The composition according to any of claims 1 to 7, wherein the pesticide comprises glyphosate or glufosinate, and additionally a further pesticide.

9. An amine alkoxylate (A) or a quaternized derivative (AQ) of the amine alkoxylate (A) according to any of claims 1 to 5.

10. The amine alkoxylate (A) or a quaternized derivative (AQ) of the amine alkoxylate (A) according to claim 9, wherein R¹, R² and R⁵ independently of one another are ethylene, ethylene and propylene, ethylene and butylene, or ethylene, propylene and butylene; wherein R³ and R⁶ are each an H; and wherein, in the amine alkoxylate (A), the total of n and m is from 2 to 40, and in its quaternized derivative (AQ) the total of n, m and p is from 3 to 80.

11. A process for the preparation of the amine alkoxylate (A) or a quaternized derivative (AQ) of the amine alkoxylate (A) according to any of claims 1 to 5, comprising the alkoxylation of 2-propylheptylamine with ethylene oxide, propylene oxide, butylene oxide or a mixture of these.

12. The process according to claim 11, wherein R¹, R² and R⁵ independently of one another are ethylene, ethylene and propylene, ethylene and butylene, or ethylene, propylene and butylene; wherein R³ and R⁶ are each an H; and wherein, in the amine alkoxylate (A), the total of n and m is from 2 to 40, and in its quaternized derivative (AQ) the total of n, m and p is from 3 to 80.

13. A method of controlling phytopathogenic fungi and/or undesired vegetation and/or undesired insect or mite attack and/or for regulating the growth of plants, wherein the composition according to any of claims 1 to 8 is allowed to act on the respective pests, their environment or the plants to be protected from the respective pest, on the soil and/or on undesired plants and/or the crop plants and/or their environment.

14. Seed, comprising the composition according to any of claims 1 to 8.

15. The use of the amine alkoxylate (A) or a quaternized derivative (AQ) of the amine alkoxylate (A) according to any of claims 1 to 5 as adjuvants in pesticide-comprising spray mixtures.

16. The use according to claim 13, wherein the adjuvant is an activity-enhancing adjuvant.

## Revendications

1. Composition comprenant un pesticide et un alcoxylate, **caractérisée en ce que** l'alcoxylate est un alcoxylate d'amine (A) 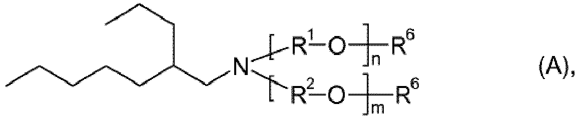 ou un dérivé quaternisé (AQ) 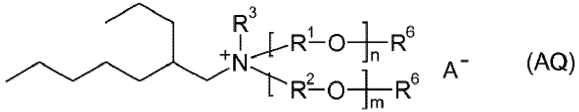 de l'alcoxylate d'amine (A),
R¹, R² et R⁵ représentant indépendamment les uns des autres éthylène, propylène, butylène ou un mélange de ceux-ci,
R³ représentant un H, -OH, -OR⁴, -[R⁵-O]ₚ-R⁶, alkyle en C₁-C₆ ou un anion oxygène,
R⁴ représentant un alkyle en C₁-C₆, alcényle en C₂-C₆ ou alcynyle en C₂-C₆,
R⁶ représentant un H, alkyle en C₁-C₆, alcényle en C₂-C₆, alcynyle en C₂-C₆, -SO₃R^{a}, -P(O)OR^{b}OR^{c}, -CH₂CO₂R^{d} ou-C(O)R^{e},
R^{a} et R^{d} représentant indépendamment l'un de l'autre un H, des cations inorganiques ou organiques,
R^{b} et R^{e} représentant indépendamment l'un de l'autre un H, des cations inorganiques ou organiques, alkyle en C₁-C₆, alcényle en C₂-C₆ ou alcynyle en C₂-C₆,
R^{e} représentant alkyle en C₁-C₂₂, alcényle en C₂-C₂₂, alcynyle en C₂-C₂₂, aryle en C₆-C₂₂, alkylaryle en C₇-C₂₂, n, m et p représentant indépendamment les uns des autres une valeur de 1 à 30,
A⁻ représentant un anion acceptable en agriculture ou, si R³ représente un anion oxygène, A⁻ n'étant pas présent.

2. Composition selon la revendication 1, dans laquelle R¹, R² et R⁵ représentent indépendamment les uns des autres éthylène, éthylène et propylène, éthylène et butylène, ou éthylène, propylène et butylène.

3. Composition selon la revendication 1 ou 2, dans laquelle A⁻ est un halogénure, un phosphate, un sulfate ou un pesticide anionique.

4. Composition selon l'une quelconque des revendications 1 à 3, dans laquelle R³ représente un H.

5. Composition selon l'une quelconque des revendications 1 à 4, dans laquelle, dans l'alcoxylate d'amine (A), la somme de n et m est de 2 à 40 et, dans son dérivé quaternisé (AQ), la somme de n, m et p est de 3 à 80.

6. Composition selon l'une quelconque des revendications 1 à 5, dans laquelle l'alcoxylate est l'alcoxylate d'amine (A).

7. Composition selon l'une quelconque des revendications 1 à 6, dans laquelle le pesticide comprend un pesticide comprenant au moins un groupe H acide.

8. Composition selon l'une quelconque des revendications 1 à 7, dans laquelle le pesticide comprend le glyphosate ou le glufosinate, et également un autre pesticide.

9. Alcoxylate d'amine (A) ou dérivé quaternisé (AQ) de l'alcoxylate d'amine (A) selon l'une quelconque des revendications 1 à 5.

10. Alcoxylate d'amine (A) ou dérivé quaternisé (AQ) de l'alcoxylate d'amine (A) selon la revendication 9, dans lequel R¹, R² et R⁵ représentent indépendamment les uns des autres éthylène, éthylène et propylène, éthylène et butylène, ou éthylène, propylène et butylène ; R³ et R⁶ représentent chacun un H ; et, dans l'alcoxylate d'amine (A), la somme de n et m est de 2 à 40 et, dans son dérivé quaternisé (AQ), la somme de n, m et p est de 3 à 80.

11. Procédé de fabrication de l'alcoxylate d'amine (A) ou d'un dérivé quaternisé (AQ) de l'alcoxylate d'amine (A) selon l'une quelconque des revendications 1 à 5, comprenant l'alcoxylation de 2-propylheptylamine avec de l'oxyde d'éthylène, de l'oxyde de propylène, de l'oxyde de butylène ou un mélange de ceux-ci.

12. Procédé selon la revendication 11, dans lequel R¹, R² et R⁵ représentent indépendamment les uns des autres éthylène, éthylène et propylène, éthylène et butylène, ou éthylène, propylène et butylène ; R³ et R⁶ représentent chacun un H ; et, dans l'alcoxylate d'amine (A), la somme de n et m est de 2 à 40 et, dans son dérivé quaternisé (AQ), la somme de n, m et p est de 3 à 80.

13. Procédé de lutte contre des champignons phytopathogènes et/ou une végétation indésirable et/ou une infestation indésirable par des insectes ou des acariens et/ou de régulation de la croissance de plantes, selon lequel la composition selon l'une quelconque des revendications 1 à 8 est laissée agir sur les nuisibles respectifs, leur habitat ou les plantes à protéger des nuisibles respectifs, le sol et/ou sur des plantes indésirables et/ou les plantes utiles et/ou leur habitat.

14. Graine contenant la composition selon l'une quelconque des revendications 1 à 8.

15. Utilisation de l'alcoxylate d'amine (A) ou d'un dérivé quaternisé (AQ) de l'alcoxylate d'amine (A) selon l'une quelconque des revendications 1 à 5 en tant qu'adjuvant dans des bouillies de pulvérisation contenant un pesticide.

16. Utilisation selon la revendication 13, dans laquelle l'adjuvant est un adjuvant augmentant l'effet.
